# EUROPEAN PATENT SPECIFICATION

(11) **EP 4 371 605 B1**
(45) Date of publication and mention of the grant of the patent: **29.10.2025**
(21) Application number: 23761415.1
(22) Date of filing: 08.06.2023
(51) Int. Cl.: A61N 1/32

(54) **PORTABLE BEAUTY INSTRUMENT AND CHARGING BASE THEREOF**
TRAGBARES SCHÖNHEITSINSTRUMENT UND LADEBASIS DAFÜR
INSTRUMENT DE BEAUTÉ PORTATIF ET SA BASE DE CHARGEMENT

(30) Priority: 01.11.2022 CN 202211358848; 01.11.2022 CN 202222905740 U; 01.11.2022 CN 202222919322 U; 02.11.2022 CN 202222919382 U; 13.10.2022 CN 202211256095; 13.10.2022 CN 202222706175 U; 28.10.2022 CN 202222876159 U; 28.10.2022 CN 202222892354 U; 30.09.2022 CN 202211217053
(43) Date of publication of application: 22.05.2024
(73) Proprietor: Shenzhen Ulike Smart Electronics Co., Ltd., Shenzhen, Guangdong 518000 (CN)
(72) Inventor: YANG, Jingjing, Guangdong 518000 (CN)
(74) Representative: Valet Patent Services Limited
(86) International application number: PCT/CN2023/099235
(87) International publication number: WO 2024/066467

(56) References cited:
- WO-A1-2014/208262
- CN-A- 109 528 049
- CN-U- 210 813 482
- CN-U- 217 470 457
- JP-A- 2017 153 811
- KR-A- 20200 069 432
- US-B2- 9 504 826

## Description

### TECHNICAL FIELD

The present disclosure relates to the technical field of electronic devices, in particular to a portable beauty device and a charging dock.

### BACKGROUND

Currently, beauty devices on the market include an operating head and a light/ electricity generating circuit. The operating head is defined with an output surface for the light/ electricity generating circuit. The light or electricity generated by the light/electricity generating circuit reaches skin and treats the skin, which can improve the skin.

The beauty device on the market includes two housings that are mutually matched. The interior of the housings is defined with a cavity housing for receiving various circuits and structural components that realize beauty functions, such as electrodes, light sources, batteries, circuit boards, etc. This will lead to a disorganized internal structure of the beauty device, low space utilization, large volume, and difficult to ensure the structural strength of the beauty device.

WO2014208262 A1 provides a beauty aid. The beauty aid (1) is constituted by a unit group that includes at least a first unit (20) and a second unit (10) which are mutually detachable from an assembly, and is generally bar-shaped when assembled. The first unit (20) is provided with a power supply means, a grip part gripped by the user, and a first connector part for connecting to the second unit (10). The second unit is provided with a function-specific applicator structure that provides beauty functions, and a second connector part for connecting to the first unit. The base (100) of the second unit (10) is formed to a flat panel shape, the second connector part is situated on one surface of the base, and the function-specific applicator structure (110) is furnished on another surface. The first unit (20) has a recessed portion (20a) designed to interlock in opposition the surface of the base (100) on the second connector part side thereof, providing an integrated appearance when assembled.

### SUMMARY

In view of the deficiencies of the related art, the present disclosure provides a portable beauty device and its charging dock. The utilization of space inside the portable beauty device of the present disclosure is improved, the size of the portable beauty device is reduced, and the structural strength can also be taken into account.

The present disclosure is set out in the appended set of claims.

In order to achieve the above, the present disclosure utilizes the following technical solutions.

A portable beauty device includes a middle frame, a rear housing, a front housing assembly, an electrode, and a processing circuit. The middle frame includes a first mounting plate extending along a first direction and a second mounting plate extending along a second direction. The rear housing at least partially surrounds the first mounting plate and defines a second accommodating space cooperatively with the first mounting plate. The front housing assembly is located on a side of the second mounting plate away from the rear housing and defines a third accommodating space cooperatively with the second mounting plate. The electrode is arranged in the third accommodating space and is at least partially exposed out of the front housing assembly. The processing circuit is arranged in the second accommodating space and is connected to the electrode to drive the electrode to discharge.

In some embodiments, the rear housing includes a first side housing and a second side housing, the first side housing is installed on a side of the first mounting plate, and the second side housing is installed the opposite side of the first mounting plate. The first side housing, the first mounting plate, and the second mounting plate cooperatively define a first accommodating space; the second side housing, the first mounting plate, and the second mounting plate cooperatively define the second accommodating space.

In some embodiments, the portable beauty device further includes a first sealing member. The first sealing member includes a first sealing strip and two second sealing strips, all of which are integrated. The first sealing strip at least partially surrounds a peripheral side of the second mounting plate to seal a gap between the first side housing and the second mounting plate, as well as a gap between the second side housing and the second mounting plate. Each second sealing strip is configured to seal either one of the gaps formed between a first lateral side of the first mounting plate and the first side housing, between the first lateral side and the second side housing, between a second lateral side of the first mounting plate opposite to the first lateral side and the first side housing, and between the second lateral side and the second side housing.

In some embodiments, each second sealing strip extends along both the front and back sides of either one of the first lateral side and the second lateral side of the first mounting plate, and contacts both the first side housing and the second side housing. Or, each second sealing strip extends along either one of opposite surfaces of the first mounting plate to seal either one of the gaps formed between a surface of the first mounting plate and the first side housing, and between the opposite surface of the first mounting plate and the second side housing.

In some embodiments, the first mounting plate includes a first plate and and two first side plates. Each first side plate is connected to either one of two opposite sides of the first plate, each first side plate has a width greater than the thickness of the first plate, each second sealing strip is arranged around the edge of either one of the first side plates. The ends of the two first side plates adjacent to the second mounting plate divide the first sealing strip into two symmetrical parts, with each part of the first sealing strip extending from one of the first side plates to another.

In some embodiments, each first side plate is defined with a first wire groove in a lateral side of the first side plate and extending along a length direction of the first side plate, a peripheral side of the second mounting plate is defined with a second wire groove extending around a circumference of the second mounting plate, and two first wire grooves of the two first side plates and the second wire groove are communicated to form a closed and three-dimensional annular groove. The first sealing strip is embedded in the second wire groove, and each second sealing strip is embedded in either one of the first wire grooves.

In some embodiments, an inner wall of the first side housing is provided with a first protruding rib that interlocks with the annular groove, an inner wall of the second side housing is provided with another first protruding rib that interlocks with the annular groove, and the first protruding ribs press the first sealing strip in the second wire groove.

In some embodiments, the front housing assembly includes a front housing and an annular housing, with the annular housing located between the front housing and the second mounting plate to connect the front housing and the second mounting plate, and a side of the front housing and/or the annular housing is defined with a concave.

In some embodiments, the portable beauty device further includes a second sealing member and a connecting rib. The second sealing member seals a gap between the annular housing and the second mounting plate. The connecting rib is connected between the first sealing strip and the second sealing member, and/or connected between the two second sealing strips.

In some embodiments, the portable beauty device further includes a bottom housing. The first mounting plate includes a first plate and two first side plates, and each first side plate is connected to either one of two opposite sides of the first plate. The bottom housing is installed on an end of the first mounting plate away from the second mounting plate, both ends of the bottom housing in a length direction of the bottom housing extend to respectively abut with either one of the two first side plate.

In some embodiments, the portable beauty device further includes a first button assembly. The middle frame further includes a third mounting plate. The third mounting plate is arranged on a side of the first mounting plate away from the second mounting plate, and the first button assembly is arranged on the third mounting plate and is at least partially exposed out of the bottom housing.

In some embodiments, the first button assembly includes a button circuit board, a key, a key housing with a semi-transparent top, and a light-emitting member. The button circuit board and the light-emitting member are connected to the processing circuit. The key housing covers the key and the light-emitting member, and the key and the light-emitting member are mounted on the button circuit board.

In some embodiments, the key housing includes a key housing cover and a key housing body. The key housing cover is integrally light transmissive. The key housing body with a light-shielded perimeter and a top defined with a light transmission hole corresponding to the light-emitting member, wherein light emitted by the light-emitting member passes through the light transmission hole and is projected from the key housing cover.

In some embodiments, a bottom of the key housing protrudes to form a second column, and a rib plate is provided in the second column. The key is inserted into the second column, the rib plate in the second column makes contact with the key housing, and the key housing above the key is capable of being pressed to activate the key.

In some embodiments, a bottom of the key housing protrudes to form a third column, a rib plate is provided in the third column, and the rib plate defines different compartments in the third column. The button circuit board is provided with at least two light-emitting members, the number of the compartments at a bottom of the third column matches the number of the light-emitting members on the button circuit board, each light-emitting member is placed in one of the compartments, and light emitted by each light-emitting member is projected from a corresponding compartment.

In some embodiments, the first button assembly includes an elastic member connected between a free end of the key housing and the bottom housing. A side of the bottom housing facing the key housing is provided with a plurality of first columns, the elastic member is defined with apertures, and each first column is inserted into one of the apertures.

In some embodiments, the portable beauty device further includes a second button assembly, wherein the first button assembly and the second button assembly are arranged on a same button circuit board. The bottom housing is defined with a first through-hole and a second through-hole, the first button assembly protrudes through the first through-hole, and the second button assembly protrudes through the second through-hole.

Another purpose of the present disclosure is to provide a charging dock of the portable beauty device. The charging dock includes a first seat body, a charging component, and a second seat body. The first seat body includes a supporting surface and a first limiting surface, and the supporting surface abuts the first limiting surface to define an angle therebetween and form a projection. The charging component is arranged on the supporting surface. The second seat body is connected to a side of the first limiting surface of the first seat body, the second seat body includes a second limiting surface, and the second limiting surface and the first limiting surface define an angle therebetween and a depression. The second limiting surface is configured to limit the front housing assembly and the electrode of the portable beauty device in a first limiting direction, the depression between the second limiting surface and the first limiting surface is configured to support a side surface of the front housing assembly, the first limiting surface is configured to limit a connection portion between the front housing assembly and the rear housing of the portable beauty device in a second limiting direction, and respective horizontal components of the first limiting direction and the second limiting direction are opposite to each other.

In some embodiments, the supporting surface is curved.

In some embodiments, the charging dock further includes a disinfection lamp. The disinfection lamp is arranged on the second limiting surface for emitting light to disinfect the portable beauty device.

In the present disclosure, the first mounting plate and the second mounting plate, which extend in two different directions respectively, are connected to form the middle frame. The middle frame is served as a mounting body of the front housing assembly and the rear housing, and defines different accommodating spaces in the housing for receiving the electrodes and the circuits respectively, which can not only improve the overall structural strength of the beauty device, but also increase utilization of space inside the beauty device and reduce the volume of the beauty device.

### BRIEF DESCRIPTION OF THE DRAWINGS

FIG. 1 is a schematic perspective view of a middle frame of a portable beauty device according to an embodiment of the present disclosure.
FIG. 2 is a schematic perspective view of a front housing of the portable beauty device of FIG. 1.
FIG. 3 is a schematic view of a circuit structure of the portable beauty device according to an embodiment of the present disclosure.
FIG. 4 is a schematic perspective view of a first sealing member of the portable beauty device according to an embodiment of the present disclosure.
FIG. 5 is an exploded view of the portable beauty device according to an embodiment of the present disclosure.
FIG. 6 is an enlarged view of FIG. 5.
FIG. 7 is another exploded view of the portable beauty device according to an embodiment of the present disclosure.
FIG. 8 is a cross-sectional view of a housing of the portable beauty device according to an embodiment of the present disclosure.
FIG. 9 is an enlarged view of FIG. 8.
FIG. 10 is another enlarged view of FIG. 8.
FIG. 11 is a schematic diagram of the overall portable beauty device according to an embodiment of the present disclosure.
FIG. 12 is a schematic view of the circuit structure of the portable beauty device according to another embodiment of the present disclosure.
FIG. 13 is a schematic perspective view of the first sealing member of the portable beauty device according to another embodiment of the present disclosure.
FIG. 14 is a schematic perspective view of a first accommodating space of the portable beauty device according to an embodiment of the present disclosure.
FIG. 15 is a schematic view of a first button assembly of the portable beauty device of FIG. 14 from one perspective.
FIG. 16 is a schematic view of the first button assembly of the portable beauty device of FIG. 14 from another perspective.
FIG. 17 is a schematic perspective view of the middle frame according to another embodiment of the present disclosure.
FIG. 18 is a schematic perspective view of a charging dock according to an embodiment of the present disclosure.
FIG. 19 is a cross-sectional view of the charging dock according to an embodiment of the present disclosure.
FIG. 20 is a schematic perspective view of a mating state of the charging dock and the portable beauty device according to an embodiment of the present disclosure.
FIG. 21 is a schematic perspective view of mounting the portable beauty device to the charging dock according to an embodiment of the present disclosure.
FIG. 22 is a schematic perspective view of the charging dock according to another embodiment of the present disclosure.
FIG. 23 is a cross-sectional view of the charging dock of FIG. 22.
FIG. 24 is a schematic view of a circuit structure of the charging dock according to an embodiment of the present disclosure.

The realization of the purpose of the present disclosure, functional features, and advantages will be further described in conjunction with the embodiments and with reference to the accompanying drawings.

### DETAILED DESCRIPTION OF THE EMBODIMENTS

In the present disclosure, terms of "set", "provided with", and "connected" should be broadly interpreted. For example, the term can refer to a fixed connection, a detachable connection, or an integral construction; the term can be a mechanical connection or an electrical connection; the term can be a direct connection or an indirect connection through an intermediate medium; the term can also refer to an internal communication between two devices, components, or parts. Those skilled in the art can understand specific meanings of the terms above in the present disclosure based on specific circumstances.

The terms "center," "longitudinal," "lateral," "length," "width," "thickness," "top," "bottom," "front," "rear," "left," "right," "vertical," "horizontal," "upper," "lower," "inner," "outer," "axial," "radial," "circumferential," and similar terms indicating orientation or positional relationships are based on the orientations or positional relationships shown in the accompanying drawings. These terms are used for the purpose of facilitating and simplifying the description of the present disclosure, and should not be construed as indicating or implying that the device or component referred to must have a specific orientation or be constructed and operated in a specific orientation. Therefore, they should not be understood as limiting the scope of the present disclosure.

Furthermore, the terms "first" and "second" are used for descriptive purposes only and should not be construed as indicating or implying relative importance or implying a specific quantity of the indicated technical features. Therefore, features designated as "first" or "second" may explicitly or implicitly include at least one instance of that feature. In the description of the present disclosure, the term "multiple" means at least two, such as two, three, etc., unless otherwise explicitly specified.

Furthermore, the terms above, in addition to indicating orientation or positional relationships, may also be used to represent other meanings. For example, the term "on" in certain contexts may also refer to an attachment or connection relationship. For those skilled in the art, the specific meanings of these terms in the present disclosure can be understood based on the specific circumstances.

In order to provide a clearer understanding of the purposes, technical solutions, and advantages of the present disclosure, further detailed explanations are provided below in conjunction with the accompanying drawings and exemplary embodiments. It should be understood that the specific embodiments described here are merely intended to explain the present disclosure and are not intended to limit the scope of the present disclosure.

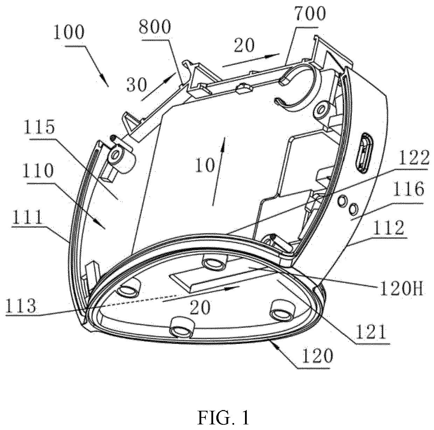

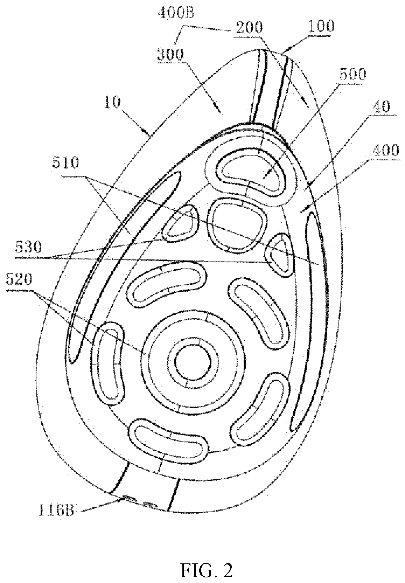

FIG. 1 is a schematic perspective view of a middle frame of a portable beauty device according to an embodiment of the present disclosure. FIG. 2 is a schematic perspective view of a front housing of the portable beauty device of FIG. 1.

Referring to FIG. 1, FIG. 2, and FIG. 8, the beauty device of the embodiment includes a middle frame 100, a first side housing 200 and a second side housing 300, a front housing assembly 40, and an electrode 500. The middle frame 100 includes a first mounting plate 110 extending along a first direction 10 and a second mounting plate 120 extending along a second direction 20. The first mounting plate 110 includes a first lateral side 111 and a second lateral side 112 opposite to the first lateral side, and a third lateral side 113 (obscured by the second mounting plate 120) connecting between the first lateral side 111 and the second lateral side 112. The second mounting plate 120 includes a first surface 121 and a second surface 122 disposed back-to-back, and the second mounting plate 120 is connected to the third lateral side 113 of the first mounting plate 110 through the second surface 122. The first side housing 200 and the second side housing 300 are respectively installed on either one of opposite sides of the first mounting plate 110. The first side housing 200, the second side housing 300, and the second mounting plate 120 cooperatively define a first accommodating space 1500 and a second accommodating space 1600 separated by the first mounting plate 110. That is, the first accommodating space 1500 is provided on a side of the first mounting plate 110 and the second accommodating space 1600 is provided on the opposite side of the first mounting plate 110. The front housing assembly 40 includes a front housing 400, and the front housing assembly 40 is arranged opposite to the second surface 122 of the second mounting plate 120 and define a third accommodating space 1700 cooperatively with the first surface 121 of the second mounting plate 120. The electrode 500 is arranged on the front housing 400, and a portion of the electrode 500 is located in the front housing 400 and at least partial the electrode 500 is exposed out of the front housing 400.

The housing 10 of the embodiment includes the first side housing 200, the second side housing 300, and the front housing assembly 40. By using the middle frame 100 as a mounting body, the first side housing 200, the second side housing 300, and the front housing assembly 40 are respectively assembled on different parts of the middle frame 100. The first side housing 200, the second side housing 300, the front housing assembly 40, and the first mounting plate 110 cooperatively form a main component of the housing of the portable beauty device. The outer surfaces of the first side housing 200, the second side housing 300, the front housing assembly 40, and the first mounting plate 110, and an outer lateral side of the second mounting plate 120 cooperatively construct an exterior surface of the portable beauty device. Such a structure may make assembly of the beauty device be convenient. And shape of the first side housing 200 and the second side housing 300 are substantially the same, so as to make the housing of the beauty device a more regular shape, allowing users to grip it more easily.

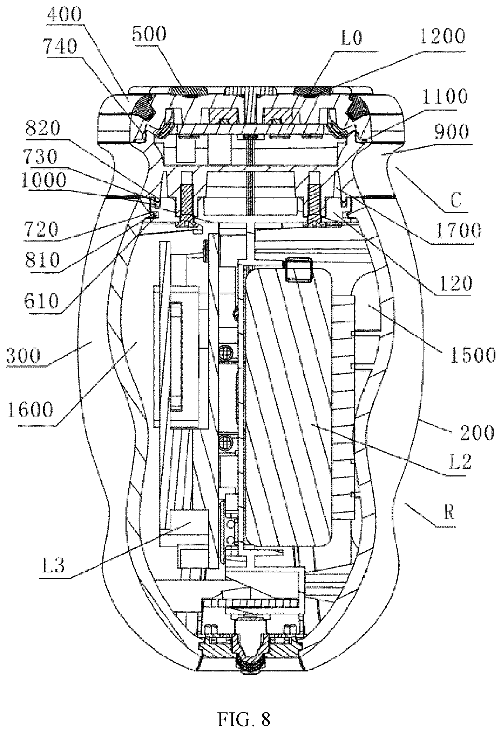

Referring to FIG. 8, which is a cross-sectional view of a housing of the portable beauty device according to an embodiment of the present disclosure. As shown in FIG. 2 and FIG. 8, the first side housing 200 and the second side housing 300 cooperatively form a side housing, also referred to rear housing 400B described later in the present disclosure. The side housing includes two opposite depressions, that is, each of the first side housing 200 and the second side housing 300 defines a depression R. Additionally, there is an annular groove C defined in the front housing assembly 40 near the side housing. The depression R and the annular groove C are ergonomic and facilitate users to grip the beauty device during use and prevent the beauty device from falling out of the hand.

In some embodiments, as shown in FIG. 1, the first direction 10 and the second direction 20 are perpendicular to each other. As an example, the first direction 10 is parallel to a central axis of the portable beauty device, and the second direction 20 is perpendicular to the central axis of the portable beauty device.

In some embodiments, the first mounting plate 110 and the second mounting plate 120 may be roughly flat boards, or structures with protrusions and indentations, or hollow structures.

In some embodiments, the third lateral side 113 of the first mounting plate 110 is connected to a middle part of the second mounting plate 120.

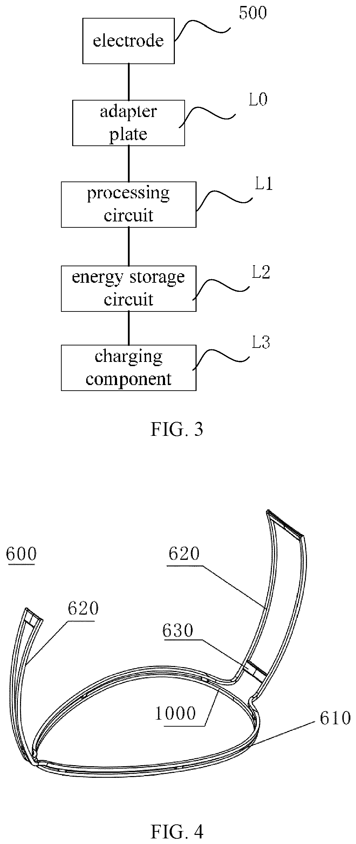

Referring to FIG. 3, in some embodiments, the portable beauty device includes a processing circuit L1, an energy storage circuit L2, a charging component L3, and an adapter plate L0, etc.. The processing circuit L1 is configured to drive the electrode 500 to discharge. The energy storage circuit L2 is configured to store energy required by the portable beauty device. The charging component L3 is configured to input energy to the energy storage circuit L2. The processing circuit L1, the energy storage circuit L2, and the charging component L3 may be arranged in either one of the first accommodating space 1500 and the second accommodating space 1600 simultaneously, or respectively arranged in the first accommodating space 1500 and the second accommodating space 1600.

For example, the energy storage circuit L2 is arranged in the first accommodating space 1500, the processing circuit L1 and the charging component L3 are arranged in the second accommodating space 1600, the adapter plate L0 is arranged in the third accommodating space 1700, and the adapter plate L0 is connected to the electrode 500. The processing circuit L1 outputs radio-frequency current and/or micro-current to the electrode 500 through the adapter plate L0, so as to perform cosmetic treatment on skin when the electrode 500 contacts the skin.

As shown in FIG. 2, in some embodiments, the electrode 500 include a first electrode 510, a second electrode 520, and a third electrode 530. The first electrode 510 is located on a connection area of an end portion and a side portion of the front housing 400 of the housing 10. The second electrode 520 is located on the end portion of the front housing 400, and the third electrode 530 is located on the end portion or the side portion of the front housing 400. The third electrode 530 generates the radio-frequency current or the micro-current, and forms a first electrode pair cooperatively with the first electrode 510 to generate current to stimulate and improve the skin. In addition, number of the second electrodes 520 is at least two, and the two second electrodes define a second electrode pair. The first electrode pair and the second electrode pair may discharge to the skin individually or cooperatively. Users can select different modes of the beauty device to control the number of the electrode pairs that discharge, thereby controlling the intensity of the beauty device.

The first electrode 510 and the second electrodes 520 may individually discharge electric current to act on the skin, which expands the range that the beauty device can irradiate. For example, users can hold a side portion of the beauty device and make end portion of the beauty device contact with the skin, and part of the second electrodes 520 and the first electrode 510 discharge. If the biological parts around the nose need to be stimulated, the beauty device may be tilted so that the first electrode 510 can in contact with the nose. For another example, if users only want to treat a small area of skin with the beauty device, the beauty device can be tilted to allow the first electrode 510 contacting with the skin to stimulate the skin.

In some embodiments, the end portion and the side portion define an angle of less than 120 degrees, and the connection area is an bending area between the end portion and the side portion. The number of the first electrodes 510 is two, both of the first electrodes 510 extend along a bending line of the bending area. Each first electrode 510 has a portion located on the end portion, a portion located on the bending line, and a remaining portion located on the side portion. Of course, ignoring the bending line, it can also be considered that a portion of the first electrode 510 is located on the end portion, and a remaining portion of the first electrode 510 is located on the side portion. For example, the angle defined between the end portion and the side portion of the beauty device may be 110 degrees or 70 degrees. In the exemplary embodiment, the angle between the end portion and the side portion is less than 120 degrees, which can fit the skin of some undulating parts better to tight the skin, such as the nose, when using the beauty device to operate beauty treatment.

During the use of the beauty device, the electrodes 500 generate micro-current that acts on the skin, which stimulates the skin to achieve lifting and firming of the skin and anti-aging effect. The first electrode 510 includes two arc-shaped electrode tips both located on the end portion and side portion of the front housing 400. By way of arc-shaped electrode tips, the first electrode 510 may fit the facial contours better, especially at depressed areas of the nasolabial folds. Therefore, it results in better effects in lifting and firming the skin.

In some other embodiments, the first electrode 510 may be a dot electrode and do not necessarily extend along the bending line of the bending area.

In some embodiments, the energy storage circuit L2 may be a capacitor, a battery, etc..

By arranging different components in different accommodating spaces of the portable beauty device, it not only prevents the affect of one component to another caused by overheating during operation, but also secures each of the components within the portable beauty device better.

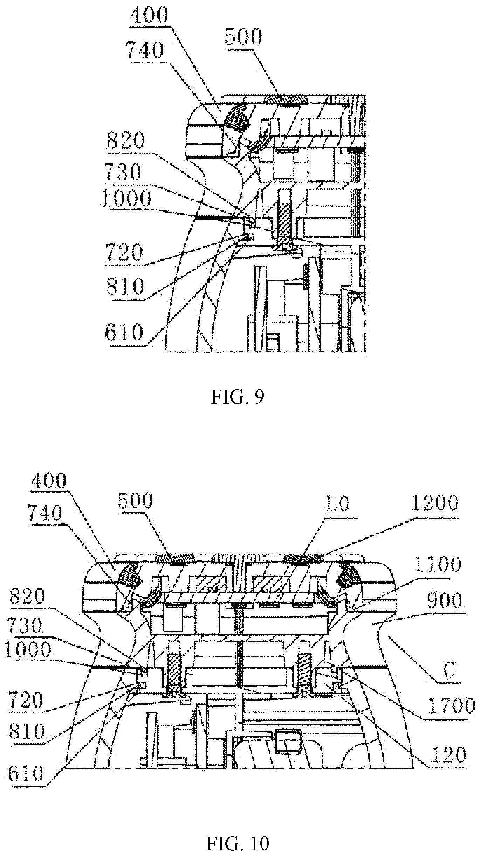

FIG. 3 is a schematic view of a circuit structure of the portable beauty device according to an embodiment of the present disclosure. FIG. 8 is a cross-sectional view of a housing of the portable beauty device according to an embodiment of the present disclosure. FIG. 10 is an enlarged view of FIG. 8.

Referring to FIG. 3, FIG. 8, and FIG. 10, in some embodiments, the processing circuit L1 works in conjunction with the electrodes 500. By providing micro-current and/or radio-frequency current to the electrodes 500 through the processing circuit L1, the skin can be stimulated. The device can also achieve effects such as rapid skin lifting, potent anti-aging, tight and plump skin, a V-shaped face in 3 minutes, and diminishing nasolabial folds by working in conjunction with a phototherapy lamp irradiating the skin.

In some embodiments, by working in conjunction with multiple components such as the processing circuit L1, the energy storage circuit L2, the charging component L3, and the electrodes 500, the charging component L3 can charge and provide energy to the portable beauty device through the energy storage circuit L2. The portable beauty device can stimulate the skin by providing micro-current and radio-frequency current to the electrodes 500 through the processing circuit L1. The device can also achieve effects such as rapid skin lifting, potent anti-aging, tight and plump skin, a V-shaped face in 3 minutes, and diminishing nasolabial folds by working in conjunction with a phototherapy lamp irradiating the skin.

FIG. 4 is a schematic perspective view of a first sealing member of the portable beauty device according to an embodiment of the present disclosure.

Referring to FIG. 1 and FIG. 4, in some embodiments, the portable beauty device further includes a first sealing member 600. The first sealing member 600 includes a first sealing strip 610 and second sealing strips 620, all of which are integrated. When the first sealing member 600 is installed on the portable beauty device, the first sealing strip 610 at least partially surrounds a peripheral side of the second mounting plate 120, and wraps around a circumferential edge of the second mounting plate 120. The first sealing strip 610 is configured to seal a gap between the first side housing 200 and the second mounting plate 120, as well as a gap between the second side housing 300 and the second mounting plate 120.

As an example, the first sealing member 600 includes two second sealing strips 620. Either one of the two second sealing strips 620 is located on either one of the opposite first lateral side 111 and the second lateral side 112 of the first mounting plate 110. One of the second sealing strips 620 extends along both front and back sides of the first lateral side 111 of the first mounting plate 110, and another second sealing strips 620 extends along both front and back sides of the second lateral side 112 of the first mounting plate 110. In other words, either one of the two second sealing strips 620 wraps around both the front and backsides of either one of the first lateral side 111 and the second lateral side 112. Each of the second sealing strips 620 contacts both the first side housing 200 and the second side housing 300. The two second sealing strips 620 are configured to seal the gaps formed between the first side housing 200 and the first lateral side 111 of the first mounting plate 110, between the first side housing 200 and the second lateral side 112 of the first mounting plate 110, between the second side housing 300 and the first lateral side 111 of the first mounting plate 110, and between the second side housing 300 and the second lateral side 112 of the first mounting plate 110, in case the first side housing 200 and the second side housing 300 are installed on the first mounting plate 110. For example, the length of the second sealing strip 620 may match the length of the corresponding first lateral side 111 and second lateral side 112 of the first mounting plate 110 to achieve a complete sealing at the joint between the first mounting plate 110 and the first side housing 200, and between the first mounting plate 110 and the second side housing 300.

In some embodiments, the first sealing member 600 may be integrated with the middle frame 100, which reduces gaps and achieves better waterproof and dustproof effects. It can also ensure a better fitness between the first sealing member 600 and the middle frame 100.

In some embodiments, the first sealing member 600 may be a three-dimensional structure that is integrally molded and has a structure and shape matching the contour of the middle frame 100. This allows for better fitting with the middle frame 100. That is, the first sealing strip 610 is looped around an edge of the second mounting plate 120, and the second sealing strips 620 are looped around edges of the first lateral side 111 and the second lateral side 112 in a width direction of the first and second lateral sides. When the first side housing 200 and the second side housing 300 are installed on opposite sides of the first mounting plate 110, the first side housing 200 and the second side housing 300 press the second sealing strips 620 and the first sealing strip 610 tightly on a surface of the first mounting plate 110. In other words, each of the first side housing 200 and the second side housing 300 presses the first sealing strip 610 on a surface of the second mounting plate 120, the first side housing 200 presses one of the second sealing strips 620 on a side surface of the first lateral side 111 of the second mounting plate 120, and the second side housing 300 presses the other of the second sealing strips 620 on a side surface of the second lateral side 112 of the second mounting plate 120 opposite to the side surface of the first lateral side 111.

Since the first sealing strip 610 can prevent water and dust from entering the first accommodating space 1500 and the second accommodating space 1600 through the gaps formed between the first side housing 200 and the second mounting plate 120 as well as between the second side housing 300 and the second mounting plate 120, and the second sealing strips 620 can prevent water and dust from entering the first accommodating space 1500 and the second accommodating space 1600 through the gaps formed between the first side housing 200 and the first lateral side 111, between the first side housing 200 and the second lateral side 112 of the first mounting plate 110, as well as between the second side housing 300 and the first lateral side 111, and between the second side housing 300 and the second lateral side 112 of the first mounting plate 110, the tight fit between the first sealing member 600 and the middle frame 100 eliminates the occurrence of surface gaps on the portable beauty device, resulting in improved waterproof and dustproof effects.

In some embodiments, as shown in FIG. 1, the first mounting plate 110 includes a first plate 115 and two first side plates 116. Each first side plate 116 is connected to either one of opposite left and right sides of the first plate 115. One of the first side plate 116 has two ends along a width direction, with both provided with the first lateral sides 111; and the other first side plate 116 has two ends along a width direction, with both provided with the second lateral sides 112.

As such, one of the first side plates 116 defines one of the first lateral sides 111 on a side of the first plate 115 and the other first lateral sides 111 on the other opposite side of the first plate 115, and the other first side plates 116 defines one of the second lateral sides 112 on a side of the first plate 115 and the other second lateral sides 112 on the other opposite side of the first plate 115. The first plate 115, the first side housing 200, and the second side housing 300 cooperatively define the first accommodating space 1500 and the second accommodating space 1600 by way of the division of the first plate 115.

In some embodiments, as shown in FIG. 4, the first sealing strip 610 is an annular sealing strip with two notches. The approximate ring-shaped first sealing strip 610 is divided into two symmetrical parts at the two notches. The two symmetrical parts include a first part and a second part, both of which are arc-shaped. Each of the second sealing strips 620 is U-shaped, and either one of the second sealing strips 620 is connected with the first sealing strip 610 at one of the two notches, forming a closed ring cooperatively with the first sealing strip 610. Each first side plate 116 of the first mounting plate 110 has a width greater than the thickness of the first plate 115. Either one of the front and back sides of the first mounting plate 110 (i.e., along a direction perpendicular to the first mounting plate 110 in FIG. 1) is provided with either one of the first lateral side 111 and the second lateral side 112. Either one of the two notches corresponds to an end of either one of the two first side plates 116 adjacent to the second mounting plate 120. The ends of the two first side plates 116 adjacent to the second mounting plate 120 divide the approximate ring-shaped first sealing strip 610 into the two symmetrical parts.

In some embodiments, when the first sealing member 600 is installed on the middle frame 100, the first sealing strip 610 is arranged around the edge of the second mounting plate 120, and the second sealing strips 620 are arranged around the edges of the first side plates 116. In other words, the two notches of the first sealing member 600 are sheltered against each of the two first side plates 116, with the first part and second part of the first sealing strip 610 extending between the two first side plates 116. That is, each part of the first sealing strip 610 extends from one of first side plates 116 to another, but on different sides of the two first side plates 116. One end of each second sealing strip 620 is connected to one end of the first part, and another end of each second sealing strip 620 extends along the edge of the first side plate 116 before connecting back to one end of the second part.

In some embodiments, as shown in FIG. 4, connecting ribs 630 are provided to connect the two sealing strips together to form the first sealing member 600. This can help reducing gaps and improving waterproof property, reducing assembly steps and increasing efficiency. Alternatively, connecting ribs 630 can also be configured to connect different parts of the two second sealing strips 620.

In some embodiments, the first mounting plate 110 may not include the first plate 115, and instead, either one of the two first side plates 116 is connected to either one of the left and right sides of the second mounting plate 120. By incorporating the second sealing strips 620, it is possible to prevent water from entering the first accommodating space 1500 and the second accommodating space 1600 through the gap formed between the first side housing 200 and the first lateral side 111, the gap formed between the first side housing 200 and the second lateral side 112, the gap formed between the second side housing 300 and the first lateral side 111, as well as the gap formed between the second side housing 300 and the second lateral side 112.

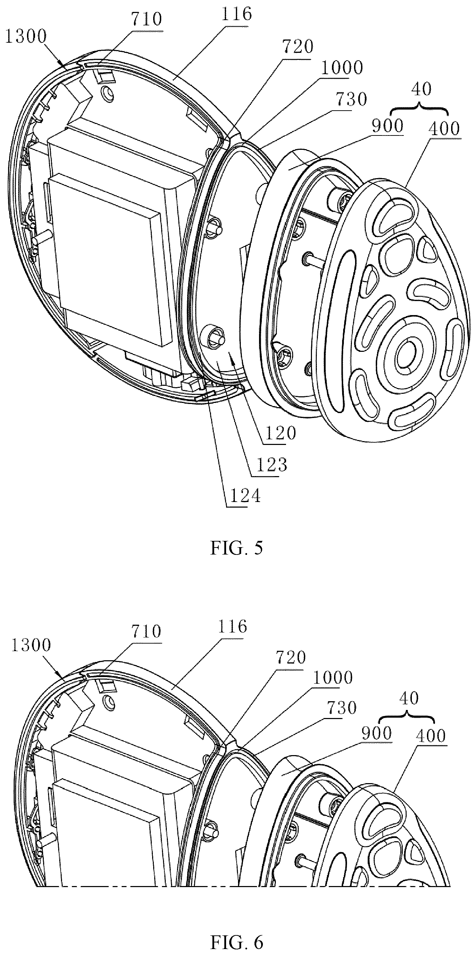

FIG. 5 is an exploded view of the portable beauty device according to an embodiment of the present disclosure. FIG. 6 is an enlarged view of FIG. 5.

Referring to FIG. 5 and FIG. 6, each of the first side plate 116 is defined with a first wire groove 710 in a lateral side of the first side plate 116 extending along a length direction of the first side plate 116. A peripheral side of the second mounting plate 120 is defined with a second wire groove 720 extending around a circumference of the second mounting plate 120. The two first wire grooves 710 of the two first side plates 116 and the second wire groove 720 of the second mounting plate 120 are communicated to form a closed and three-dimensional annular groove. The first sealing strip 610 is embedded in the annular groove. Either one of the second sealing strips 620 is embedded in either one of the first wire grooves 710, and the first sealing strip 610 is embedded in the second wire groove 720.

By providing wire grooves on a surface of the middle frame 100, the first sealing strip 610 can be fixed before assembling the beauty device. Then, after assembling the first side housing 200, the second side housing 300, and the front housing assembly 40 on the middle frame 100, the gaps formed between the first side housing 200 and the middle frame 100, and between the second side housing 300 and the middle frame 100 can be sealed simultaneously. There is no need to set sealing components for each gap individually. This not only improves the convenience of assembling the beauty device, but also ensures sealing, waterproofing, and dustproofing effects of the beauty device. Additionally, it provides a smooth and aesthetically pleasing surface for the beauty device while the waterproof property is taken into account.

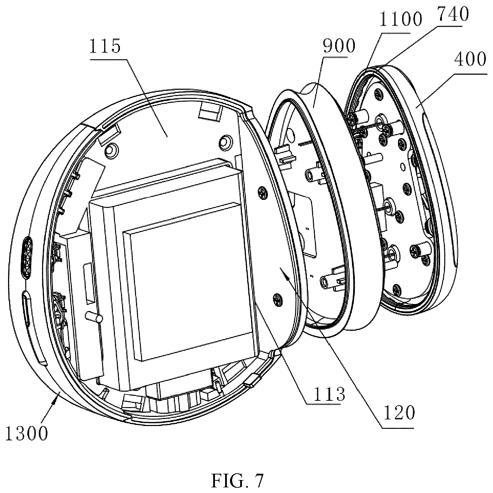

FIG. 7 is another exploded view of the portable beauty device from a different perspective than that of FIG. 5.

Referring to FIG. 4, FIG. 5, and FIG. 7, the portable beauty device includes an annular housing 900, a second sealing member 1000, and a third sealing member 1100. In addition to the front housing 400, the front housing assembly 40 also includes the annular housing 900. The front housing 400, the annular housing 900, and the second mounting plate 120 cooperatively define the third accommodating space 1700. An outer surface of the annular housing 900 defines the annular groove C.

The annular housing 900 is located between the front housing 400 and the second mounting plate 120 to connect the front housing 400 and the second mounting plate 120. The second sealing member 1000 extends along an edge of the second mounting plate 120 away from the first mounting plate 110, and forms an annular shape. The second sealing member 1000 is configured to seal a gap formed between the annular housing 900 and the second mounting plate 120. The third sealing member 1100 can be considered as a part of the front housing assembly 40. The third sealing member 1100 extends along the edge of the front housing 400 towards the annular housing 900, and forms an annular shape. The third sealing member 1100 is configured to seal a gap formed between the annular housing 900 and the front housing 400.

In the exemplary embodiment, the second sealing member 1000 seals the gap formed between the annular housing 900 and the second mounting plate 120, and the third sealing member 1100 seals the gap formed between the annular housing 900 and the front housing 400. The second sealing member 1000 and the third sealing member 1100 prevent water from entering the space between the front housing 400 and the second mounting plate 120, thus avoiding the damage to circuit boards and other components which could disrupt the normal operation of the beauty device.

Understandably, in some other embodiments, the second sealing member 1000 and the third sealing member 1100 can also be omitted.

FIG. 8 is a cross-sectional view of a housing of the portable beauty device according to an embodiment of the present disclosure. FIG. 9 is an enlarged view of FIG. 8.

Referring to FIG. 5 and FIG. 7 to FIG. 9, in some embodiments, the second mounting plate 120 includes a second plate 123 and a second side plate 124. One surface of the second side plate 124 is connected to a peripheral side of the second plate 123 to form an annular plate. The annular plate is located on a side of the second plate 123 away from the first mounting plate 110. A surface of the annular plate away from the first mounting plate 110 is defined with a third wire groove 730 that extends along an edge of the annular plate. The second sealing member 1000 is arranged in the third wire groove 730. A surface of the front housing 400 towards the annular housing 900 defines a notch 740 extending along an inner edge of the front housing 400. The third sealing member 1100 is arranged in the notch 740.

When the front housing 400 is installed on one side of the annular housing 900, the third sealing member 1100 seals the gap formed between the front housing 400 and the annular housing 900. When the annular housing 900 is installed on the second mounting plate 120, the second sealing member 1000 seals the gap formed between the annular housing 900 and the second side plate 124 of the second mounting plate 120.

By arranging the third sealing member 1100 within the notch 740 of the front housing 400 adjacent to a peripheral edge of the annular housing 900, it can prevent water from entering the gap formed between the front housing 400 and the annular housing 900. Additionally, it ensures a tighter assembly between the front housing 400 and the annular housing 900.

In some other embodiments, the second mounting plate 120 may not include the second plate 123.

In some embodiments, the connecting ribs of the portable beauty device can be connected between the first sealing strip 610 and the second sealing member 1000, and/or connected between different portions of the second sealing strips 620. The connection of the connecting ribs prevents the first sealing strip 610 detaching from the second sealing member 1000, and ensures the waterproofness of the beauty device.

FIG. 10 is another enlarged view of FIG. 8. Referring to FIG. 8 and FIG. 10, in some embodiments, an inner wall of the first side housing 200 is provided with a first protruding 810 that interlocks with the annular groove, and an inner wall of the second side housing 300 is also provided with a first protruding rib 810 that interlocks with the annular groove. The first side housing 200 and the second side housing 300 are assembled with the first mounting plate 110 and the second mounting plate 120 by interlocking the first protruding ribs 810 with the annular groove. The first protruding ribs 810 can press the first sealing strip 610 in the annular groove, ensuring a sealing effect.

A surface of the annular housing 900 towards the second mounting plate 120 is provided with a second protruding rib 820 that interlocks with the third wire groove 730. The annular housing 900 is assembled with the second mounting plate 120 by interlocking the second protruding rib 820 with the third wire groove 730. An end of the annular housing 900 towards the front housing 400 is defined with a card slot extending along an outer sidewall of the annular housing 900. The card slot includes a bottom and a sidewall. Either one of the bottom and the sidewall abuts with either one of the two surfaces (i.e., the inner ring surface and an end surface) of the third sealing member 1100.

As such, both the first side housing 200 and the second side housing 300 are assembled with the first mounting plate 110 and the second mounting plate 120 through the first protruding ribs 810. The annular housing 900 is assembled with the second mounting plate 120 by interlocking the second protruding rib 820 with the third wire groove 730. Additionally, the end of the annular housing 900 towards the front housing 400 is defined with the card slot extending along the outer sidewall of the annular housing 900. The assembly between the front housing 400 and the annular housing 900 is made tighter by the presence of the third sealing member 1100 inside the card slot.

In some other embodiments of the present disclosure, the portable beauty device further includes a fourth sealing member 1200. The front housing 400 is defined with through-holes. The fourth sealing member 1200 is fitted onto the electrodes 500, and the electrodes 500 are inserted into the through-holes and sealed with the front housing 400 by the fourth sealing member 1200. The fourth sealing member 1200 prevents water from entering the front housing 400 through the gap formed between the electrodes 500 and the front housing 400.

In some other embodiments of the present disclosure, the electrodes 500 and the front housing 400 are integrated molded, and the electrodes 500 are exposed on a surface of the front housing 400. Therefore, there is no gap formed between the electrodes 500 and the front housing 400, such that there is no need of sealing members for waterproof.

In some embodiments of the present disclosure, as shown in FIG. 1, FIG. 2, and FIG. 11, the housing 10 of the portable beauty device also includes a bottom housing 1300. The bottom housing 1300 may be installed on an end of the first mounting plate 110 away from the second mounting plate 120. Both ends of the bottom housing 1300 in a length direction of the bottom housing 1300 extend to respectively abut with either one of the two first side plates 116. After installation, the bottom housing 1300 covers a portion of the first mounting plate 110 that is opposite to the third lateral side 113, and the bottom housing 1300 is positioned between the first side housing 200 and the second side housing 300. Either one of the ends of the bottom housing 1300 in a width direction abuts with either one of the first side housing 200 and the second side housing 300. Surfaces of the bottom housing 1300, the first side housing 200, the second side housing 300, and the first side plates 116 of the first mounting plate 110 are aligned, forming a smooth transition on a surface of the beauty device.

In some other embodiments of the present disclosure, the portable beauty device includes a light therapy lamp that is located between the front housing 400 and the second mounting plate 120. The front housing 400 includes a light transmissive portion, light emitted by the light therapy lamp is projected from the light transmissive portion. The light therapy lamp emits light through the light transmissive portion, and the light can accelerate blood circulation in the targeted skin area, promote metabolism, enhance cellular activity, and aid in tissue repair. It can also inhibit sebum secretion, suppress skin inflammation, and regulate keratinization.

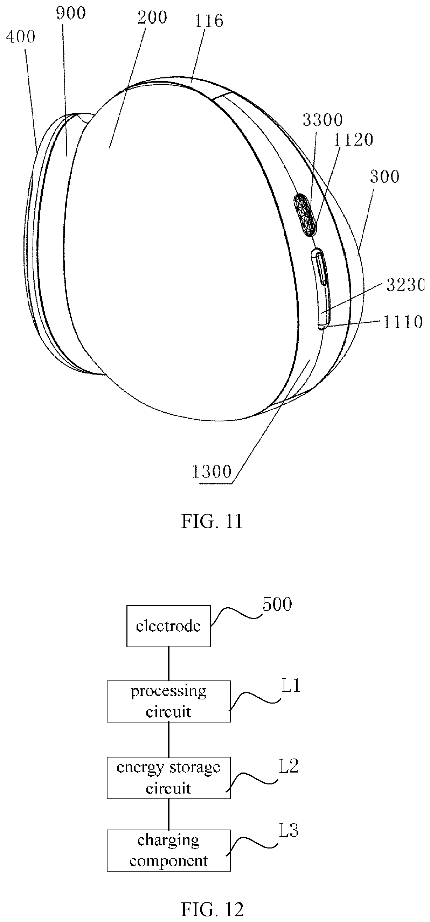

FIG. 12 is a schematic view of the circuit structure of the portable beauty device according to another embodiment of the present disclosure.

Referring to FIG. 1, FIG. 8, and FIG. 12, the portable beauty device includes a processing circuit L1 and an energy storage circuit L2. The processing circuit L1 is arranged in the second accommodating space 1600 and connected to the electrodes 500. The energy storage circuit L2 is arranged in the first accommodating space 1500 and configured to provide power to the processing circuit L1. Additionally, a region of the second mounting plate 120 corresponding to the first accommodating space 1500 is defined with a wire threading hole 120H. The processing circuit L1 is connected to the electrodes 500 through the wire threading hole 120H.

The energy storage circuit L2 provides power to the processing circuit L1, and the processing circuit L1 is connected to the electrodes 500 through the wire threading hole 120H. This allows the processing circuit L1 to control an operation of the electrodes 500. Depending on different operating modes, the processing circuit L1 controls the electrodes 500 to output various power or electric current, thereby achieving different functions and effects.

In some other embodiments of the present disclosure, the charging component L3 is connected to the energy storage circuit L2 and is located on the middle frame 100 or arranged in the first accommodating space 1500 and second accommodating space 1600. The charging component L3 includes a wireless charging coil or a charging interface.

The charging component L3 provides power to the energy storage circuit L2. When the energy storage circuit L2 reaches a certain energy level, the charging component L3 will stop delivering energy to the energy storage circuit L2. Similarly, when the energy level of the energy storage circuit L2 drops below a certain threshold, the charging component L3 will deliver energy to the energy storage circuit L2.

FIG. 12 is a schematic view of the circuit structure of the portable beauty device according to another embodiment of the present disclosure. Referring to FIG. 1 and FIG. 12, the first sealing member 1400 includes a first sealing strip 1410 and a second sealing strip 1420. When the first sealing member 1400 is installed on the portable beauty device, the first sealing strip 1410 at least partially surrounds a peripheral side of the second mounting plate 120 and wraps around a circumferential edge of the second mounting plate 120, to seal the gap between the first side housing 200 and the second mounting plate 120, as well as the gap between the second side housing 300 and the second mounting plate 120.

As an example, the first sealing member 1400 includes two second sealing strips 1420. In contrast to the first sealing member 1400 as shown in FIG. 4, either one of the two second sealing strips 1420 is located on either one of two opposite surfaces of the first mounting plate 110. Either one of the two second sealing strips 1420 extends along the first lateral side 111 and the second lateral side 112 of the first mounting plate 110. One of the second sealing strip 1420 seals the gaps formed between the first side housing 200 and the first side plates 116 of the first mounting plate 110, and contacts with the first side housing 200. The other second sealing strip 1420 seals the gaps formed between the second side housing 300 and the first side plates 116, and contacts with the second side housing 300.

The first sealing strip 1410 is divided into two symmetrical parts: the first part and the second part, both of which are curved. One of the second sealing strip 1420 extends along an edge of a surface of the first mounting plate 110, starting from an end of the first part of the first sealing strip 1410, and connecting back to another end of the first part. Another second sealing strip 1420 extends along an edge of another surface of the first mounting plate 110, starting from an end of the second part of the first sealing strip 1410, and connecting back to another end of the second part. In other words, either one of the second sealing strips 1420 connects to both two ends of either one of the first part and the second part. Moreover, the second sealing strips 1420 are embedded in the wire grooves of the two first side plates 116 and the bottom housing 1300.

As such, the first sealing member 1400 is obtained by assembling two sealing strips, and either one of the two sealing strips surrounds a contour of either one of the two surfaces of the second mounting plate 120. Either one of the two sealing strips defines the notch at either one of the regions where the second mounting plate 120 connects to the first side plates 116. A portion of either one of the sealing strips extending along either one of edges of the second mounting plate 120 can be considered as the first sealing strip 1410. Meanwhile, each of the sealing strip extends from different surfaces of the second mounting plate 120 along either one of the first lateral side 111 and the second lateral side 112 of the first mounting plate 110, and connects to each other to form the two second sealing strips 1420.

In some embodiments, the connecting ribs 1430 are configured to connect the two sealing strips together and form the first sealing member 1400. This can reduce assembly steps of the sealing member and improve assembly efficiency.

By arranging the first sealing member 1400, the beauty device in the embodiment can prevent water from entering the first accommodating space 1500 and the second accommodating space 1600 through the gap between the first side housing 200 and the second mounting plate 120, the gap between the second side housing 300 and the second mounting plate 120, the gap between the first side housing 200 and the first lateral side 111, the gap between the first side housing 200 and the second lateral side 112, the gap between the second side housing 300 and the first lateral side 111, and the gap between the second side housing 300 and the second lateral side 112.

In some other embodiments of the present disclosure, the portable beauty device includes two accommodating spaces. The side housing surrounds the first mounting plate 110, and the side housing and the first mounting plate 110 cooperatively define the first accommodating space 1500. The front housing 400 and the second mounting plate 120 define the third accommodating space 1700, and the electrodes 500 are located in the front housing 400. For example, the portable beauty device includes the middle frame 100, the side housing, the front housing 400, and the electrodes 500. The middle frame 100 includes the first mounting plate 110 extending along the first direction 10 and the second mounting plate 120 extending along the second direction 20. The first mounting plate 110 includes the first lateral side 111 and the second lateral side 112 away from each other, as well as the third lateral side 113 connecting the first lateral side 111 and the second lateral side 112. The second mounting plate 120 includes a first surface 121 and a second surface 122 away from each other, and the second mounting plate 120 is connected to the third lateral side 113 of the first mounting plate 110 through the second surface 122. The side housing at least partially surrounds the first mounting plate 110, defining the first accommodating space 1500. The front housing assembly 40 is located away from the second surface 122 of the second mounting plate 120, defining the third accommodating space 1700 cooperatively with the second mounting plate 120. The electrodes 500 are located on the front housing assembly 40 and are at least partially exposed out of the front housing assembly 40.

In comparison to the previous embodiments, the exemplary embodiment includes only one side housing. The side housing may be a collective term for the first side housing 200 and the second side housing 300, may include only the first side housing 200 or the second side housing 300, may be a structure in which the first side housing 200 and the second side housing 300 are connected integrately, or may be a modified structure of the first side housing 200 or the second side housing 300.

In some embodiments, the processing circuit L1, the energy storage circuit L2, and the charging component L3 may be arranged in the first accommodating space 1500. The electrodes 500 and the adapter plate L0 are arranged in the third accommodating space 1700, and the adapter plate L0 is connected to the electrodes 500. The processing circuit L1 outputs radio-frequency current or micro-current to the electrodes 500 through the adapter plate L0.

It should be understood that in some other embodiments, the processing circuit L1 can be arranged in either one of the first accommodating space 1500, the second accommodating space 1600, and the third accommodating space 1700.

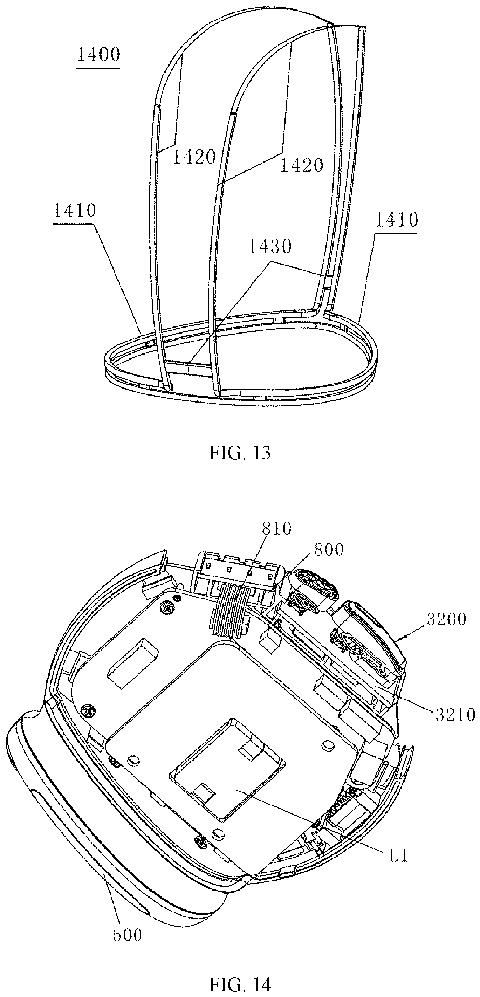

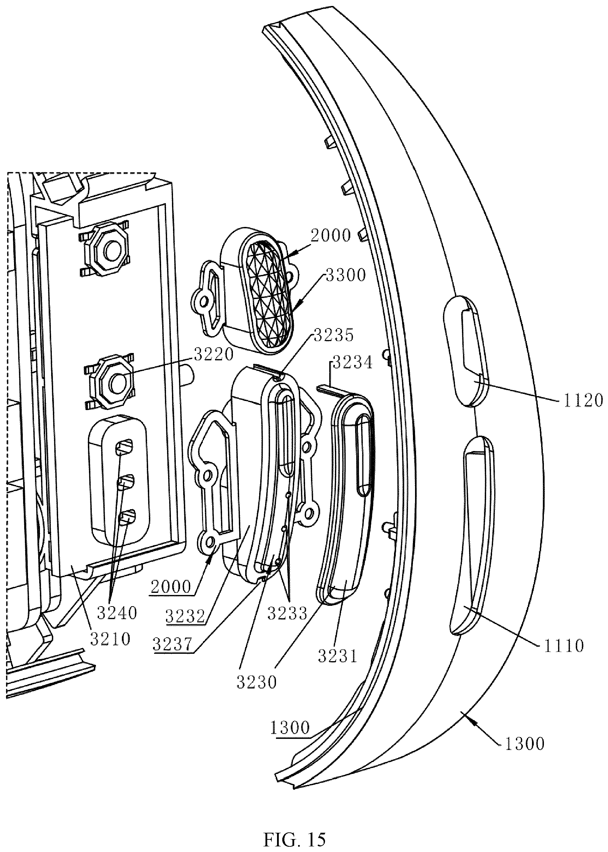

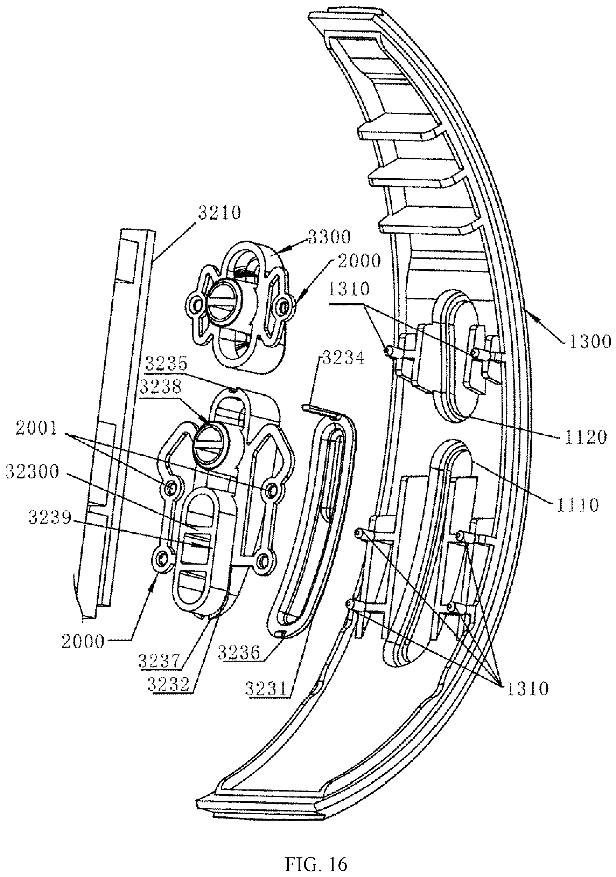

FIG. 14 is a schematic perspective view of a first accommodating space of the portable beauty device according to an embodiment of the present disclosure. FIG. 15 is a schematic view of a first button assembly of the portable beauty device of FIG. 14 from one perspective. FIG. 16 is a schematic view of the first button assembly of the portable beauty device of FIG. 14 from another perspective.

Referring to FIG. 14 to FIG. 16, in some embodiments, the portable beauty device includes the housing 10, the electrodes 500, the processing circuit L1, and a first button assembly 3200. The electrodes 500 are at least partially exposed on a surface of the housing 10 to make contact with the skin. The processing circuit L1 is located in the housing 10 and electrically connected to the electrodes 500. The first button assembly 3200 is at least partially exposed on the surface of the housing 10. For example, a portion of top of the first button assembly 3200 is at least partially exposed on the surface of the housing 10.

By operating the first button assembly 3200, users can switch the beauty device to different working modes or power levels. When the users operate the first button assembly 3200, the first button assembly 3200 sends a signal to the processing circuit L1. The processing circuit L1, based on the signal received, controls electric current and/or voltage output to the electrodes 500, so that the electrodes 500 can switch to corresponding working mode or power level.

As an example, the first button assembly 3200 includes a button circuit board 3210, a key 3220, a key housing 3230, and at least two light-emitting members 3240. The button circuit board 3210 is connected to the processing circuit L1. The light-emitting members 3240 are connected to the button circuit board 3210 and/or the processing circuit L1 to indicate working state. The key 3220 is located opposite to the button circuit board 3210, and the key housing 3230 covers the key 3220 and the light-emitting members 3240. The key 3220 and the light-emitting members 3240 are mounted on the button circuit board 3210. At least a portion of top of the key housing 3230 includes a light transmissive portion, and at least a portion of side walls of the key housing 3230 includes a light-shielded portion.

In some embodiments, at least the top portion of the key housing 3230 is a light transmissive portion.

In some embodiments, a bottom of the key housing 3230 protrudes to form a second column 3238 and a third column 3239. The second column 3238 is provided with a rib plate 32300 which defines different compartments in the second column 3238. The third column 3239 is provided with a rib plate 32300 which defines different compartments in the third column 3239.

After assembly, the key 3220 may be embedded into the second column 3238, and the rib plate in the second column 3238 makes contact with the key housing 3230. Pressing the key housing 3230 above the key 3220 can trigger the key 3220. The number of the compartments at bottom of the third column 3239 matches the number of the light-emitting members 3240 on the button circuit board 3210. Light emitted by each light-emitting member 3240 can project from a corresponding compartment.

With such a configuration described above, the second column 3238 below the key housing 3230 can secure the key housing 3230 onto the key 3220. The rib plate in the second column 3238 contacts with the key housing 3230. When users press a corresponding area of the key housing 3230 above the key 3220, the key housing 3230 is depressed downward and triggers the key 3220 through the rib plate in the second column 3238. Therefore, the users can trigger the key 3220 by pressing the key housing 3230 above the key 3220.

Meanwhile, the third column 3239 below the key housing 3230 can secure the key housing 3230 onto the light-emitting members 3240. The rib plate defines the third column 3239 into different compartments, with the number of the compartments corresponding to the number of the light-emitting members 3240. Each light-emitting member 3240 is placed in one of the compartments. As a result, the light emitted by each light-emitting member 3240 can be projected from the corresponding compartment, effectively focusing the light and preventing light leakage.

In the current related technology, buttons (the key 3220) and indicator lights (the light-emitting members 3240) of beauty device are independently arranged, resulting in a poor structure. In the present disclosure, by way of combining the key 3220 and the light-emitting members 3240 for indicating working status together, the structure is optimized and relatively simplified.

When users press the key 3220 of the first button assembly 3200, the beauty device can switch its working mode and/or power level based on the button circuit board 3210 corresponding to the key 3220. The button circuit board 3210 sends key information generated by the corresponding key 3220 to the processing circuit L1. At the same time, the button circuit controls the light-emitting members 3240 to emit light. Different key information corresponds to different light patterns. For example, in a first power level for a lifting mode, only one light-emitting member 240 emits red light, and in a second power level for a radio-frequency mode, two light-emitting members 240 emit blue light. Simultaneously, the processing circuit L1 controls the beauty device to switch to the corresponding working mode and/or power level based on the key information.

Furthermore, in some embodiments, the key housing 3230 consists of a key housing cover 3231 and a key housing body 3232. Except for the top of the key housing body 3232 is defined with a light transmission hole 3233 corresponding to the light-emitting member 3240 of the first button assembly 3200, the remaining key housing body 3232 is not light transmissive, which can prevent light leakage. The key housing cover 3231 is integrally light transmissive.

In the exemplary embodiment, the key housing cover 3231 covers a surface of the key housing body 3232. The light emitted by the light-emitting members 3240 can pass through the corresponding light transmission hole 3233, and then be visible through the key housing cover 3231, allowing users to see the illumination status of the light-emitting members 3240.

In some embodiments, as shown in FIG. 15 and FIG. 16, a top of a back portion of the key housing cover 3231 includes a limiting column 3234 that corresponds to a guide slot 3235 on a top portion of the key housing body 3232. Additionally, a lower portion of the key housing cover 3231 includes a positioning block 3236 that corresponds to a notch 3237 in the lower portion of the key housing body 3232.

A top portion of the key housing cover 3231 is provided with the limiting column 3234, and a lower portion of the key housing cover 3231 is provided with the positioning block 3236. The top portion of the key housing body 3232 defines the guide slot 3235, and the lower portion of the key housing body 3232 defines the notch 3237. By inserting the positioning block 3236 of the key housing cover 3231 into the notch 3237 of the key housing body 3232, and inserting the limiting column 3234 of the key housing cover 3231 into the guide slot 3235 of the key housing body 3232, the key housing body 3232 and the key housing cover 3231 can be securely fixed together.

When the key 3220 is pressed and triggered, the button circuit board 3210 below the key 3220 receives the information and controls the light-emitting members 3240 above the button circuit board 3210 to emit different lights. The number and positions of the light-emitting members 3240 correspond to the number and positions of the light transmission holes 3233 of the key housing body 3232. As a result, the light emitted by the light-emitting members 3240 will pass through the corresponding light transmission holes 3233, and can be viewed through the light transmissive key housing cover 3231 to observe the light transmission from the light transmission holes 3233 in the key housing body 3232.

Therefore, when observing the light transmission from the light transmission holes 3233 of the key housing body 3232 through the transparent key housing cover 3231, the key housing cover 3231and the key housing body 3232 can effectively reduce the brightness of the light emitted by the light-emitting members 3240, which avoids an over bright light. This allows users to clearly view the light emitted by the light-emitting members 3240, while also maintaining a soft and gentle light.

In some embodiments, the key housing 3230 defines a light guide channel in an area corresponding to the light-emitting members 3240, and the periphery of the light guide channel is light shielded. As an example, the key housing cover 3231 and the key housing body 3232 define protruded arc-shaped strips at the location corresponding to the light-emitting members 3240, which can help to focus the light. As such, the light guide channel provides the space to allow the light emitted by each light-emitting member 3240 to pass through each compartment of the third column 3239 below the key housing 3230, then through the corresponding light transmission holes 3233 of the key housing body 3232, and finally through the light transmissive key housing cover 3231. The rest of the key housing body 3232, except for the light transmission holes 3233, is not light transmissive (the periphery of the key housing body 3232 is light shielded). Therefore, in the exemplary embodiment, the light emitted by the light-emitting members 3240 is emitted through the light guide channel, which avoids the light from excessive brightness, and also makes the light be more focused when illuminating. This allows users to clearly view the light emitted by the light-emitting members 3240 through the key housing 3230.

In some embodiments, the top of the key housing 3230 is semi-transparent, configured to shield the structure from outside view when the light-emitting members 3240 are not emitting light, and to transmit at least a portion of the light when the light-emitting members 3240 are emitting light. Therefore, when the light-emitting members 3240 does not emit light, there is no light coming out of the top of the key housing 3230, which avoids interfering with the users' eyes and ensures an aesthetically pleasing appearance of the beauty device. When the light-emitting members 3240 are emitting light, the top of the key housing 3230 transmits part of the light. As an example, the top surface of the key housing 3230 may be electroplated or coated to reduce the brightness of the light and provide a high-end feel without causing visual discomfort.

In some embodiments, the key 3220 and the key housing 3230 may be integrally molded, allowing for more sensitive key triggering. The integrated structure enhances the overall quality of the device and reduces the gaps between the key 3220 and the key housing 3230, thus improving the sealing performance and the waterproof and dustproof performance.

In the exemplary embodiment, different working modes or power levels can be switched through the button assembly. This allows for the control of micro-current and radio-frequency current generated by the electrodes 500 to stimulate the skin. Additionally, methods such as using phototherapy lamps to illuminate the skin can be employed to improve the skin condition. These methods can achieve at least one of the beauty effects, such as quick lifting, powerful anti-aging, firming and toning, 3-minute V-face, reduction of nasolabial folds, sculpting the eye area, strengthening the skin barrier, and achieving smooth and radiant skin.

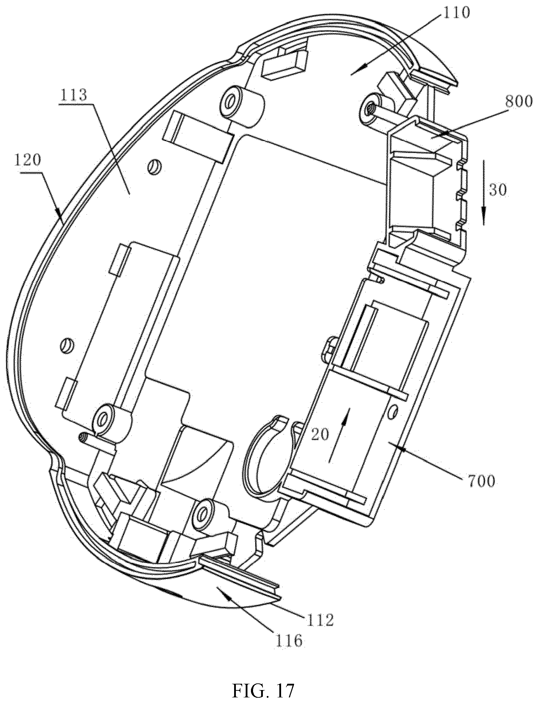

FIG. 17 is a schematic perspective view of the middle frame according to another embodiment of the present disclosure.

As shown in FIG. 1 and FIG. 17, the middle frame 100 also includes a third mounting plate 700. The third mounting plate 700 is located on a side of the first mounting plate 110 opposite to the third lateral side 113, and extends along the second direction 20. The first button assembly 3200 is located on a side of the third mounting plate 700 opposite to the second mounting plate 120.

Further referring to FIG. 14, since the first button assembly 3200 and the electrodes 500 are respectively arranged on the two ends of the portable beauty device and assembled in opposite orientations, during operating the portable beauty device, users can use the portable beauty device without accidentally triggering or pressing buttons, which prevents unintended switching of the working modes and/or power levels. This ensures a user-friendly experience of better usability.

Referring to FIG. 11, the bottom housing 1300 defines a first through-hole 1110 to expose the key housing 3230, with the key housing 3230 protruding through the first through-hole 1110.

In some embodiments, the first button assembly 3200 may be exposed outside of the bottom housing 1300 in various forms. For example, the top of the key housing 3230 is exposed outside of the bottom housing 1300; or, the button of the first button assembly 3200 is exposed outside of the bottom housing 1300, with the indicator lights embedded in the bottom housing 1300 and user viewing the number of indicators in the illuminated state through the transparent component or hole in the bottom housing 1300; or, both the button and indicator lights of the first button assembly 3200 are embedded in the bottom housing 1300.

As such, users can control the power on and/or off of the beauty device, or switch between different working modes and power levels, by pressing the first button assembly 3200 protruding through the first through-hole 1110.

Referring to FIG. 14 and FIG. 17, the portable beauty device also includes a fourth mounting plate 800 and a light-emitting circuit board 810. The fourth mounting plate 800 is located on a side of the first mounting plate 110 opposite to the third lateral side 113, and extends along a third direction 30. The light-emitting circuit board 810 is located on a side of the fourth mounting plate 800 opposite to the second mounting plate 120. A side of the light-emitting circuit board 810 towards the bottom housing 1300 is provided with several indicator lights.

As such, the fourth mounting plate 800 is adjacent to the third mounting plate 700, and the fourth morning plate 800 and the third morning plate 700 cooperatively define an angle therebetween. This allows a display structure and a button control structure to be located adjacent to a periphery of a circular or approximately circular housing, such that users can see the corresponding light indication while operating the portable beauty device, which facilitates the use for users.

The first direction 10, the second direction 20, and the third direction 30 are all different from each other. The bottom housing 1300 is provided with light transmissive patterns at the position corresponding to the indicator lights, with the number of the light transmissive patterns matching the number of the indicator lights. The indicator lights are configured to indicate different beauty modes.

The light emitted by the plurality of indicator lights passes through the bottom housing 1300, allowing users to see how many indicator lights are illuminated. This design also helps preventing the light emitted by the indicator lights from being too bright and harsh to the eyes. By weakening the light, it creates a softer and more gentle illumination for users to observe.

In some embodiments, as shown in FIG. 15 and FIG. 16, the portable beauty device further includes a second button assembly 3300. The second assembly 3300 is arranged on the button circuit board 3210.

By arranging the first button assembly 3200 and the second button assembly 3300 on a same button circuit board 3210, it is possible to save costs, facilitate assembly, and simplify the structure of the circuit board. In some embodiments, the first button assembly 3200 and the second button assembly 3300 can also be separately arranged on different button circuit boards.

The light-emitting circuit board 810 is installed on the fourth mounting plate 800, and the light emitted by the indicator lights on the light-emitting circuit board 810 will be projected through the bottom housing 1300. When users open and/or close the beauty device or switch different working modes and power levels through the first button assembly 3200 and the second button assembly 3300, the current working mode and working status of the beauty device can be determined by the light emitted by the indicator lights in the bottom housing 1300. For example, when the users press and hold the second button assembly 3300 for a long time and reach a preset time, the beauty device can be turned on and/or off. When the beauty device is turned on, the second button assembly 3300 emits white light, and when the beauty device is turned off, the second button assembly 3300 does not emit light. Or, when the beauty device is turned on, a default working mode is a skin lifting mode, and the second button assembly 3300 emits white light. When the users switch the working mode to a radio frequency mode, the second button assembly 3300 emits red light. Or, when the working mode is the skin lifting mode and the second button assembly 3300 emits white light, if a default gear is set to a first gear, only one of the light-emitting members 3240 of the first button assembly 3200 emits light, and also only one of the indicator lights of the light-emitting circuit board 810 emits light. When the users press the first button assembly 3200 to adjust the gear to a second gear, two of the light-emitting members 3240 of the first button assembly 3200 emit lights, and also two of the indicator lights of the light-emitting circuit board 810 emit lights.

Further referring to FIG. 16, an end of the key housing 3230 is connected to a side of the first through-hole 1110, and another end of the key housing 3230 is a free end. The first button assembly 3200 includes an elastic component 2000, which is connected between the free end of the key housing 3230 and the bottom housing 1300.

In some embodiments, a side of the bottom housing 1300 towards the key housing 3230 includes a plurality of first columns 1310. The elastic member 2000 defines with apertures 2001, and each first column 1310 is inserted into the apertures 2001 of the elastic member 2000 to secure the first button assembly 3200 with the bottom housing 1300.

Similarly, the bottom housing 1300 is defined with a second through-hole 1120, and the second button assembly 3300 protrudes through the second through-hole 1120. The bottom housing 1300 and the second button assembly 3300 are also secured by the first columns 1310 and the plurality of apertures 2001 of the elastic member 2000.

Therefore, users can control the beauty device to turn on and/or turn off or switch different working modes and gears, through the first button assembly 3200 protruding from the first through-hole 1110 and the second button assembly 3300 protruding from the second through-hole 1120.

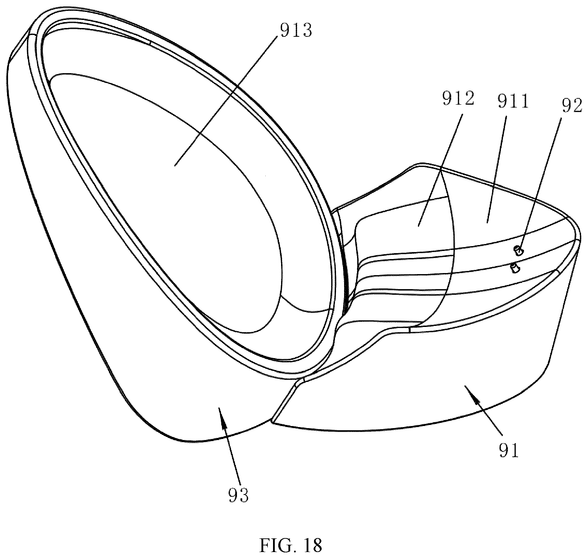

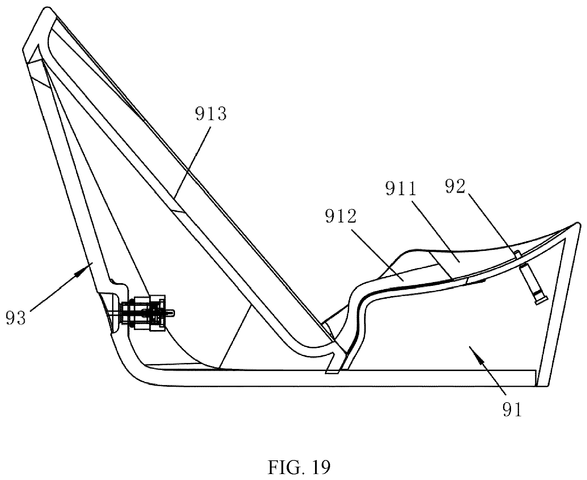

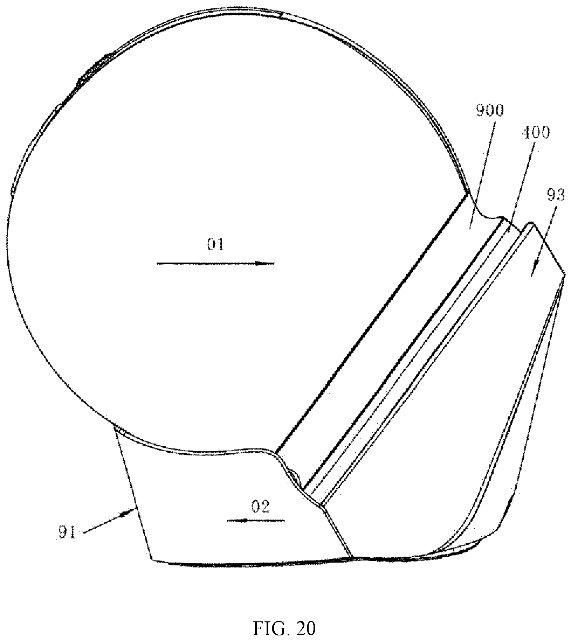

FIG. 18 is a schematic perspective view of a charging dock according to an embodiment of the present disclosure. FIG. 19 is a cross-sectional view of the charging dock of FIG. 18. FIG. 20 is a schematic perspective view of a mating state of the charging dock and the portable beauty device according to an embodiment of the present disclosure.

Referring to FIG. 18 to FIG. 20, the present disclosure also provides a charging dock, which includes a first seat body 91, a charging component 92, and a second seat body 93. The first seat body 91 includes a supporting surface 911 and a first limiting surface 912. The supporting surface 911 abuts the first limiting surface 912 to define an angle therebetween and form a projection. The charging component 92 is arranged on the supporting surface 911. The second seat body 93 is connected to a side of the first limiting surface 912 of the first seat body 91, and the second seat body 93 includes a second limiting surface 913. The second limiting surface 913 and the first limiting surface 912 define an angle therebetween and a depression. The depression between the second limiting surface 913 and the first limiting surface 912 is configured to support a side surface of the front housing assembly 40.

The second limiting surface 913 is configured to limit the portable beauty device in a first limiting direction 01, such as limiting the operation head of the beauty device (i.e., the front housing assembly 40 and the electrodes 500) in the first limiting direction 01. The first limiting surface 912 is configured to limit a neck portion of the portable beauty device (the neck portion is the part where the front housing assembly 40 is connected to the rear housing 400B, such as the side surface of the front housing 400 and/or the side surface of the annular housing 900) in a second limiting direction 02. Respective horizontal components of the first limiting direction 01 and the second limiting direction 02 are opposite to each other.

In case users need to charge the portable beauty device, the portable beauty device may be charged by inserting an end of the front housing assembly 40 with the electrodes 500 into the charging dock parallel to or inclined to a limiting direction of the first limiting surface 912, and aligning charging electrodes 116B on the first side plate 116 with the charging component 92 to charge and store energy for the portable beauty device. Further, the depression of the first limiting surface 912 fits precisely with the curvature of the side surface of the front housing assembly 40, and the supporting surface 911 supports the end face of the portable beauty device.

In some embodiments, an angle defined by the first seat body 91 and the second seat body 93 is greater than 90 degrees. The second limiting surface 913 is an inclined surface inclined in the horizontal direction.

The angle defined by the first seat body 91 and the second seat body 93 is the same as an angle defined by the front housing 400 and the first side plate 116 of the portable beauty device. Additionally, the second limiting surface 913 is an inclined surface that allows for a more stable placement of the portable beauty device on the charging dock, preventing the portable beauty device from tipping over on the charging dock.

In the exemplary embodiment, the portable beauty device is charged through the charging dock. The beauty device can either control the electrodes to generate micro-current and radio-frequency currents to stimulate and improve the skin, or to illuminate the skin by using the phototherapy lamps to achieve at least one of the various beauty effects such as quick lifting, powerful anti-aging, firming and toning, 3-minute V-face, reduction of nasolabial folds, sculpting the eye area, strengthening the skin barrier, and achieving smooth and radiant skin.

Referring to FIG. 19 to FIG. 21, the second limiting surface 913 is a concave surface, and size of the concave surface in a direction perpendicular to the horizontal direction is larger than size of a surface of the operating head of the portable beauty device. A region below the concave surface is a partially conformal surface of the operating head, with size and shape matching the size and shape of the surface of the operating head of the portable beauty device.

Therefore, the front housing 400 and the electrodes 500 exposed on the front housing 400 can both be embedded into the second limiting surface 913. The second limiting surface 913 acts as a protective casing for the front housing 400, preventing debris of the air from depositing on the operating head of the portable beauty device during periods of non-use. This helps to keep the operating head of the portable beauty device clean and prevent growth of bacteria.

In the exemplary embodiment, the first limiting surface 912 is a concave surface and serves as a partially conformal surface of the neck portion of the portable beauty device. Therefore, the concave surface of the first limiting surface 912 precisely matches the curvature of the side surface of the front housing 400. Additionally, the supporting surface 911 is an arc surface and serves as a partially conformal surface of a side portion of the portable beauty device. As a result, the supporting surface 911 precisely matches the curvature of the side surface of the first side plate 116 of the portable beauty device.

Therefore, when the portable beauty device is correctly placed on the charging dock, the side surface of the first side plate 116 of the portable beauty device is embedded and fitted with the supporting surface 911 of the first seat body 91 of the charging dock, and the operating head of the portable beauty device is embedded and fitted with the second limiting surface 913 of the second seat body 93. In some embodiments, the charging circuit is located in the first seat body 91 or the second seat body 93, and is connected to the charging component 92. Thus, the charging circuit converts an AC power input from the charging dock into an DC power, and transfers the DC power to the charging component 92. When the portable beauty device is correctly placed on the charging dock, the charging electrodes 116B on the first side plate 116 of the portable beauty device corresponds to the charging component 92 on the supporting surface 911. The DC power is transmitted by the charging component 92 and received by the charging electrodes 116B of the portable beauty device, to store the electrical energy in the energy storage circuit L2 in the portable beauty device.

When power stored in the energy storage circuit L2 inside the portable beauty device exceeds a preset power value, the charging dock will not charge the portable beauty device. When the power stored in the energy storage circuit L2 is lower than the preset power value, the charging dock will charge the portable beauty device. Additionally, the charging dock will stop charging the portable beauty device when the power stored in the portable beauty device reaches the preset power value.

Therefore, by charging the portable beauty device through the charging dock, the charging circuit 1401 can be arranged in the charging dock, which enhances the portability of charging for users. The users do not need to charge the portable beauty device by connecting it with a data cable or any other means, but simply place the portable beauty device on the charging dock, then the charging dock automatically charges the portable beauty device.

In some embodiments, the charging component 92 can be either conductive pins or conductive coils.

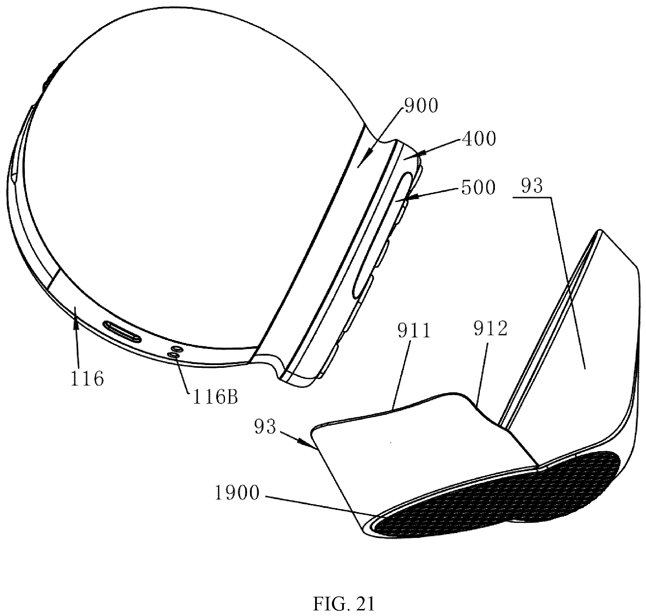

In the exemplary embodiment, a magnetic suction component is located on the first seat body 91 to attract the portable beauty device. When users place the portable beauty device on the first seat body 91, there may be positional deviations. However, the magnetic suction component corrects the deviation in position and angle through a force of magnetism, automatically adjusting the portable beauty device to a correct placement position during the placement process. In some embodiments, as shown in FIG. 21, the charging dock includes an anti-slip pad 1900, which is arranged under the first seat body 91 and the second seat body 93 to increase an adhesion of a bottom surface of the charging dock.

In case the charging dock is placed on a smooth surface, in order to prevent the charging dock from sliding easily on the smooth surface because a friction between the charging dock and the smooth surface is too small, the anti-slip pad 1900 can be provided under the first seat body 91 and the second seat body 93 to increase the friction between the charging dock and the smooth surface. When the portable beauty device is placed on the charging dock, the charging dock will receive the push force from the portable beauty device. By arranging the anti-slip pad 1900 under the first seat body 91 and the second seat body 93, the friction between the charging dock and the flat surface may partially cushion the push force, so that the charging dock is less likely to tip over.

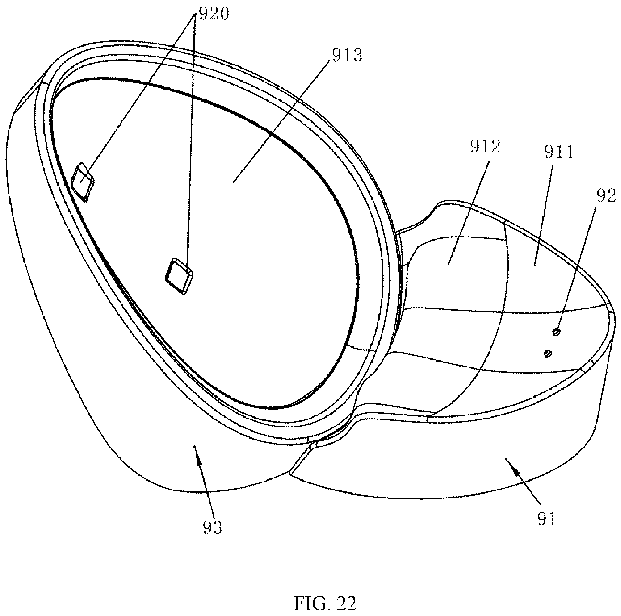

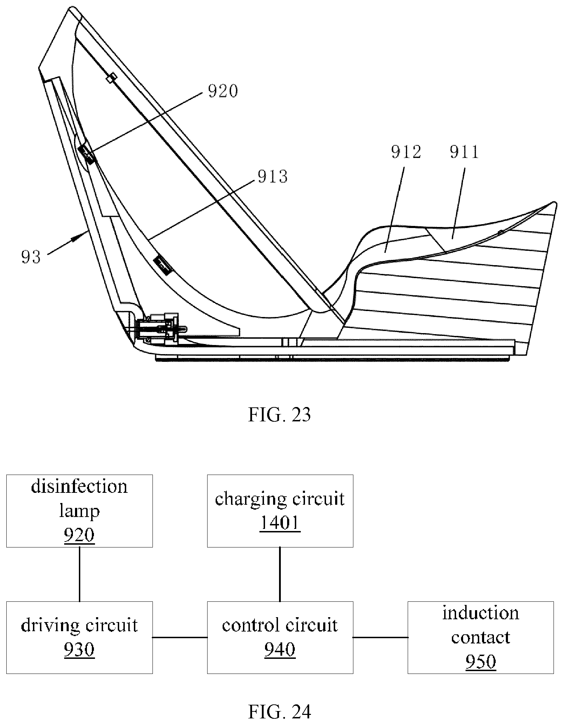

As shown in FIG. 22 to FIG. 24, another embodiment of the charging dock is illustrated. The charging dock includes a disinfection lamp 920, a driving circuit 930, a control circuit 940, and an induction contact 950. The disinfection lamp 920 is located on the second limiting surface 913 of the second seat body 93, and light emitted from the disinfection lamp 920 is configured to disinfect the front housing 400 of the portable beauty device. The driving circuit 930 is electrically connected to the disinfection lamp 920. The control circuit 940 is electrically connected to both the driving circuit 930 and the charging circuit 1401, and sends a first control signal to control the driving circuit 930 and a second control signal to control the charging circuit 1401. The induction contact 950 is arranged corresponding to the supporting surface, the first limiting surface 912, or the second limiting surface 913, and is electrically connected to the control circuit 940. The induction contact 950 sends a start signal to the control circuit 940. When the portable beauty device triggers the induction contact 950 of the charging dock, the control circuit 940 receives the start signal from the induction contact 950, and sends the first control signal to control the driving circuit 930 and the second control signal to control the charging circuit 1401. The first control signal is configured to control the driving circuit 930 to turn on the disinfection lamp 920. The second control signal is configured to control the charging circuit 1401 to charge the portable beauty device.

During the use of the portable beauty device, users are not in a sterile environment and there may be dust and bacteria in the air, or after contacting with the skin or the use of gel, the surface of the operation head of the portable beauty device may become contaminated with various substances and bacteria. When the operation head of the portable beauty device come into contacts with the skin, the bacteria on the operation head may cause acne and inflammation of the skin. The disinfection lamp 920 can irradiate ultraviolet light onto the operation head of the portable beauty device to kill the bacteria present on it, thus keeping the operation head of the portable beauty device hygienic and preventing the growth of the bacteria.

In some embodiments, the light emitted by the disinfection lamp 920 may be one and/or a combination of the colors selected from a group consisting of red, green, blue, and yellow.

Therefore, when the side surface of the first side plate 116 of the portable beauty device is embedded and fitted with the supporting surface 911 of the first seat body 91 of the charging dock, and the operating head of the portable beauty device is embedded and fitted with the second limiting surface 913 of the second seat body 93, the charging electrodes 116B of the first side plate 116 of the portable beauty device corresponds to the charging component 92 on the supporting surface 911, and the induction contact 950 receives the induction by way of pressure sensing or distance sensing. The induction contact 950 then sends the start signal to the control circuit 940. After receiving the start signal from the induction contact 950, the control circuit 940 sends the first control signal to control the driving circuit 930 and the second control signal to control the charging circuit 1401. The first control signal is configured to control the driving circuit 930 to activate the disinfection lamp 920, and the second control signal is configured to control the charging circuit 1401 to charge the portable beauty device.

In some embodiments, users can also control the disinfection lamp 920 to turn on and/or turn off through the buttons on the charging dock. This allows the users to independently control startup of the disinfection lamp 920, control disinfection time length of the disinfection lamp 920, and perceive and determine current progress of the disinfection.

Referring to FIG. 3, FIG. 8 and FIG. 21, the portable beauty device includes the front housing 40, a plurality of electrodes 500, the rear housing 400B, the annular housing 900, the processing circuit L1, the energy storage circuit L2, and the charging component L3. The front housing 400 is configured to contact the skin. The plurality of electrodes 500 are arranged on the front housing 400. The rear housing 400B includes the first side housing 200 and the second side housing 300. The rear housing 400B is configured for gripping. The annular housing 900 is located between the front housing 400 and the second mounting plate 1220 to connect the front housing 400 and the rear housing 400B. The processing circuit L1 is located inside the rear housing 400B and is connected to the plurality of electrodes 500. The energy storage circuit L2 is located inside the rear housing 400B and provides power to the processing circuit L1. The charging component L3 is located inside the rear housing 400B and is connected to the energy storage circuit L2.

In an exemplary embodiment, the charging component L3 may be either conductive coils or charging contact exposed on a surface of the rear housing 400B.

The processing circuit L1, the energy storage circuit L2, and the charging component L3 are all arranged inside the rear housing 400B. The processing circuit L1 controls the operation of the electrodes 500, the energy storage circuit L2 provides power to the processing circuit L1, and the charging component L3 supplies power to the energy storage circuit L2. When energy stored in the energy storage circuit L2 reaches a certain level, the charging component L3 stops supplying energy to the energy storage circuit L2. Similarly, when energy stored in the energy storage circuit L2 drops below a certain threshold, the charging component L3 starts supplying energy to the energy storage circuit L2.

In an exemplary embodiment, a diameter of the annular housing 900 gradually decreases and then increases along a direction of an axis of the annular housing 900. There is an angle defined between any two of the end surface of the front housing 400, partial surface of the annular housing 900, and the surface of the rear housing 400B defining the charging contacts.

The above description is only specific embodiments of the present disclosure. It should be noted that, for those skilled in the art, various modifications and refinements can be made without departing from the scope of the present disclosure. Such modifications and refinements should also be considered within the scope of protection of the present disclosure.

## Claims

1. A portable beauty device comprising: a front housing assembly (40), a rear housing (400B), a middle frame (100), an electrode (500), and a processing circuit (L1);
the middle frame (100) comprising a first mounting plate (110) extending along a first direction (10);
the rear housing (400B) at least partially surrounding the first mounting plate (110) and defining a second accommodating space (1600) cooperatively with the first mounting plate (110);
the electrode (500) being at least partially exposed out of the front housing assembly (40); and
the processing circuit (L1) being arranged in the second accommodating space (1600) and connected to the electrode (500) to drive the electrode (500) to discharge;
the middle frame (100) further comprises a second mounting plate (120) extending along a second direction (20) perpendicular to the first direction (10);
the front housing assembly (40) is located on a side of the second mounting plate (120) away from the rear housing (400B) **characterised in that** said front housing assembly (40) defines a third accommodating space (1700) cooperatively with the second mounting plate (120) for receiving the electrode (500); and
the electrode (500) is further arranged in the third accommodating space (1700).

2. The portable beauty device of claim 1, wherein,
the rear housing (400B) comprises a first side housing (200) and a second side housing (300), the first side housing (200) is installed on a side of the first mounting plate (110), and the second side housing (300) is installed on the opposite side of the first mounting plate (110);
the first side housing (200), the first mounting plate (110), and the second mounting plate (120) cooperatively define a first accommodating space (1500); the second side housing (300), the first mounting plate (110), and the second mounting plate (120) cooperatively define the second accommodating space (1600).

3. The portable beauty device of claim 2, further comprising a first sealing member (600, 1400), wherein the first sealing member (600, 1400) comprises a first sealing strip (610, 1410) and two second sealing strips (620, 1420), all of which are integrated;
the first sealing strip (610, 1410) at least partially surrounds a peripheral side of the second mounting plate (120) to seal a gap between the first side housing (200) and the second mounting plate (120), as well as a gap between the second side housing (300) and the second mounting plate (120);
each second sealing strip (620, 1420) is configured to seal either one of the gaps formed between a first lateral side (111) of the first mounting plate (110) and the first side housing (200), between the first lateral side (111) and the second side housing (300), between a second lateral side (112) of the first mounting plate (110) opposite to the first lateral side (111) and the first side housing (200), and between the second lateral side (112) and the second side housing (300).

4. The portable beauty device of claim 3, wherein each second sealing strip (620, 1420) extends along both the front and back sides of either one of the first lateral side (111) and the second lateral side (112) of the first mounting plate (110), and contacts both the first side housing (200) and the second side housing (300);
or, each second sealing strip (620, 1420) extends along either one of opposite surfaces of the first mounting plate (110) to seal either one of the gaps formed between a surface of the first mounting plate (110) and the first side housing (200), and between the opposite surface of the first mounting plate (110) and the second side housing (300).

5. The portable beauty device of claim 3, wherein the first mounting plate (110) comprises a first plate (115) and two first side plates (116), each first side plate (116) is connected to either one of two opposite sides of the first plate (115), each first side plate (116) has a width greater than the thickness of the first plate (115), each second sealing strip (620, 1420) is arranged around the edge of either one of the first side plates (116);
the ends of the two first side plates (116) adjacent to the second mounting plate (120) divide the first sealing strip (610, 1410) into two symmetrical parts, with each part of the first sealing strip (610, 1410) extending from one of the first side plates (116) to another.

6. The portable beauty device of claim 5, wherein each first side plate (116) is defined with a first wire groove (710) in a lateral side of the first side plate (116) and extending along a length direction of the first side plate (116), a peripheral side of the second mounting plate (120) is defined with a second wire groove (720) extending around a circumference of the second mounting plate (120), and two first wire grooves (710) of the two first side plates (116) and the second wire groove (720) are communicated to form a closed and three-dimensional annular groove;
the first sealing strip (610, 1410) is embedded in the second wire groove (720), and each second sealing strip (620, 1420) is embedded in either one of the first wire grooves (710);
wherein an inner wall of the first side housing (200) is provided with a first protruding rib (810) that interlocks with the annular groove, an inner wall of the second side housing (300) is provided with another first protruding rib (810) that interlocks with the annular groove, and the first protruding ribs (810) press the first sealing strip (610, 1410) in the second wire groove (720).

7. The portable beauty device of claim 3, wherein the front housing assembly (40) comprises a front housing (400) and an annular housing (900), with the annular housing (900) located between the front housing (400) and the second mounting plate (120) to connect the front housing (400) and the second mounting plate (120), and a side of the front housing (400) and/or the annular housing (900) is defined with a concave;
wherein the portable beauty device further comprises:
a second sealing member (1000), which seals a gap between the annular housing (900) and the second mounting plate (120); and
a connecting rib (630, 1430), connected between the first sealing strip (610, 1410) and the second sealing member (1000), and/or connected between the two second sealing strips (620, 1420).

8. The portable beauty device of claim 1, further comprising a bottom housing (1300), wherein the first mounting plate (110) comprises a first plate (115) and two first side plates (116), each first side plate (116) is connected to either one of two opposite sides of the first plate (115);
the bottom housing (1300) is installed on an end of the first mounting plate (110) away from the second mounting plate (120), both ends of the bottom housing (1300) in a length direction of the bottom housing (1300) extend to respectively abut with either one of the two first side plates (116).

9. The portable beauty device of claim 8, further comprising a first button assembly (3200), wherein the middle frame (100) further comprises a third mounting plate (700);
the third mounting plate (700) is arranged on a side of the first mounting plate (110) away from the second mounting plate (120), and the first button assembly (3200) is arranged on the third mounting plate (700) and is at least partially exposed out of the bottom housing (1300).

10. The portable beauty device of claim 9, wherein the first button assembly (3200) comprises a button circuit board (3210), a key (3220), a key housing (3230) with a semi-transparent top, and a light-emitting member (3240); the button circuit board (3210) and the light-emitting member (3240) are connected to the processing circuit (L1);
the key housing (3230) covers the key (3220) and the light-emitting member (3240), and the key (3220) and the light-emitting member (3240) are mounted on the button circuit board (3210).

11. The portable beauty device of claim 10, wherein the key housing (3230) comprises:
a key housing cover (3231), which is integrally light transmissive; and
a key housing body (3232), with a light-shielded perimeter and a top defined with a light transmission hole (3233) corresponding to the light-emitting member (3240), wherein light emitted by the light-emitting member (3240) passes through the light transmission hole (3233) and is projected from the key housing cover (3231).

12. The portable beauty device of claim 10, wherein a bottom of the key housing (3230) protrudes to form a second column (3238), and a rib plate (32300) is provided in the second column (3238);
the key (3220) is inserted into the second column (3238), the rib plate (32300) in the second column (3238) makes contact with the key housing (3230), and the key housing (3230) above the key (3220) is capable of being pressed to activate the key (3220);
or,
wherein a bottom of the key housing (3230) protrudes to form a third column (3239), a rib plate (32300) is provided in the third column (3239), and the rib plate (32300) defines different compartments in the third column (3239); the button circuit board (3210) is provided with at least two light-emitting members (3240), the number of the compartments at a bottom of the third column (3239) matches the number of the light-emitting members (3240) on the button circuit board (3210), each light-emitting member (3240) is placed in one of the compartments, and light emitted by each light-emitting member (3240) is projected from a corresponding compartment.

13. The portable beauty device of claim 10, wherein the first button assembly (3200) comprises an elastic member (2000) connected between a free end of the key housing (3230) and the bottom housing (1300);
a side of the bottom housing (1300) facing the key housing (3230) is provided with a plurality of first columns (1310), the elastic member (2000) is defined with apertures (2001), and each first column (1310) is inserted into one of the apertures (2001);
or,
wherein the portable beauty device further comprises a second button assembly (3330), and the first button assembly (3200) and the second button assembly (3330) are arranged on a same button circuit board (3210);
the bottom housing (1300) is defined with a first through-hole (1110) and a second through-hole (1120), the first button assembly (3200) protrudes through the first through-hole (1110), and the second button assembly (3330) protrudes through the second through-hole (1120).

14. A charging dock of the portable beauty device as claimed in any one of claims 1-13, wherein the charging dock comprises:
a first seat body (91) comprising a supporting surface (911) and a first limiting surface (912), the supporting surface (911) abutting the first limiting surface (912) to define an angle therebetween and form a projection;
a charging component (L3) arranged on the supporting surface (911); and
a second seat body (93) connected to a side of the first limiting surface (912) of the first seat body (91), the second seat body (93) comprising a second limiting surface (913), the second limiting surface (913) and the first limiting surface (912) defining an angle therebetween and a depression;
wherein the second limiting surface (913) is configured to limit the front housing assembly (40) and the electrode (500) of the portable beauty device in a first limiting direction (01), the depression between the second limiting surface (913) and the first limiting surface (912) is configured to support a side surface of the front housing assembly (40), the first limiting surface (912) is configured to limit a connection portion between the front housing assembly (40) and the rear housing (400B) of the portable beauty device in a second limiting direction (02), and respective horizontal components of the first limiting direction (01) and the second limiting direction (02) are opposite to each other.

15. The charging dock of claim 14, wherein the supporting surface (911) is curved; and/or, wherein the charging dock further comprises a disinfection lamp (920) arranged on the second limiting surface (913) for emitting light to disinfect the portable beauty device.

## Patentansprüche

1. Tragbares Schönheitsinstrument, umfassend: eine vordere Gehäusebaugruppe (40), ein hinteres Gehäuse (400B), einen Mittelrahmen (100), eine Elektrode (500) und eine Verarbeitungsschaltung (L1);
wobei der Mittelrahmen (100) eine erste Montageplatte (110) umfasst, die sich entlang einer ersten Richtung (10) erstreckt;
wobei das hintere Gehäuse (400B) zumindest teilweise die erste Montageplatte (110) umgibt und zusammen mit der ersten Montageplatte (110) einen zweiten Aufnahmeraum (1600) definiert;
wobei die Elektrode (500) zumindest teilweise aus der vorderen Gehäusebaugruppe (40) heraus freiliegt; und
wobei die Verarbeitungsschaltung (L1) in dem zweiten Aufnahmeraum (1600) angeordnet und mit der Elektrode (500) verbunden ist, um die Elektrode (500) zur Entladung anzutreiben;
wobei der Mittelrahmen (100) ferner eine zweite Montageplatte (120) umfasst, die sich entlang einer zweiten Richtung (20) erstreckt, die senkrecht zur ersten Richtung (10) verläuft;
wobei sich die vordere Gehäusebaugruppe (40) auf einer Seite der zweiten Montageplatte (120) befindet, die von dem hinteren Gehäuse (400B) abgewandt ist, **dadurch gekennzeichnet, dass** die vordere Gehäusebaugruppe (40) zusammen mit der zweiten Montageplatte (120) einen dritten Aufnahmeraum (1700) zum Aufnehmen der Elektrode (500) definiert;
wobei die Elektrode (500) ferner im dritten Aufnahmeraum (1700) angeordnet ist.

2. Tragbares Schönheitsinstrument nach Anspruch 1, wobei
das hintere Gehäuse (400B) ein erstes Seitengehäuse (200) und ein zweites Seitengehäuse (300) umfasst, wobei das erste Seitengehäuse (200) an einer Seite der ersten Montageplatte (110) angebracht ist und das zweite Seitengehäuse (300) an der gegenüberliegenden Seite der ersten Montageplatte (110) angebracht ist;
das erste Seitengehäuse (200), die erste Montageplatte (110) und die zweite Montageplatte (120) zusammen einen ersten Aufnahmeraum (1500) definieren; das zweite Seitengehäuse (300), die erste Montageplatte (110) und die zweite Montageplatte (120) zusammen den zweiten Aufnahmeraum (1600) definieren.

3. Tragbares Schönheitsinstrument nach Anspruch 2, ferner umfassend ein erstes Dichtungselement (600, 1400), wobei das erste Dichtungselement (600, 1400) einen ersten Dichtungsstreifen (610, 1410) und zwei zweite Dichtungsstreifen (620, 1420) umfasst, die alle integriert sind;
wobei der erste Dichtungsstreifen (610, 1410) zumindest teilweise eine periphere Seite der zweiten Montageplatte (120) umgibt, um einen Spalt zwischen dem ersten Seitengehäuse (200) und der zweiten Montageplatte (120) sowie einen Spalt zwischen dem zweiten Seitengehäuse (300) und der zweiten Montageplatte (120) abzudichten;
wobei jeder zweite Dichtungsstreifen (620, 1420) konfiguriert ist, um einen der Spalte abzudichten, die zwischen einer ersten seitlichen Seite (111) der ersten Montageplatte (110) und dem ersten Seitengehäuse (200), zwischen der ersten seitlichen Seite (111) und dem zweiten Seitengehäuse (300), zwischen einer zweiten seitlichen Seite (112) der ersten Montageplatte (110), die der ersten seitlichen Seite (111) gegenüberliegt, und dem ersten Seitengehäuse (200), sowie zwischen der zweiten seitlichen Seite (112) und dem zweiten Seitengehäuse (300) gebildet werden.

4. Tragbares Schönheitsinstrument nach Anspruch 3, wobei sich jeder zweite Dichtungsstreifen (620, 1420) entlang sowohl der Vorder- als auch der Rückseite einer der ersten Seitenfläche (111) und der zweiten Seitenfläche (112) der ersten Montageplatte (110) erstreckt und sowohl das erste Seitengehäuse (200) als auch das zweite Seitengehäuse (300) berührt;
oder, wobei sich jeder zweite Dichtungsstreifen (620, 1420) entlang einer der gegenüberliegenden Flächen der ersten Montageplatte (110) erstreckt, um jeweils einen der zwischen einer Fläche der ersten Montageplatte (110) und dem ersten Seitengehäuse (200) sowie zwischen der gegenüberliegenden Fläche der ersten Montageplatte (110) und dem zweiten Seitengehäuse (300) gebildeten Spalte abzudichten.

5. Tragbares Schönheitsinstrument nach Anspruch 3, wobei die erste Montageplatte (110) eine erste Platte (115) und zwei erste Seitenplatten (116) umfasst, wobei jede erste Seitenplatte (116) mit einer der beiden gegenüberliegenden Seiten der ersten Platte (115) verbunden ist, wobei jede erste Seitenplatte (116) eine Breite aufweist, die größer ist als die Dicke der ersten Platte (115), wobei jeder zweite Dichtungsstreifen (620, 1420) um die Kante einer der ersten Seitenplatten (116) angeordnet ist;
wobei die Enden der beiden ersten Seitenplatten (116), die an der zweiten Montageplatte (120) benachbart sind, den ersten Dichtungsstreifen (610, 1410) in zwei symmetrische Teile teilen, wobei sich jeder Teil des ersten Dichtungsstreifens (610, 1410) von einer der ersten Seitenplatten (116) zur anderen erstreckt.

6. Tragbares Schönheitsinstrument nach Anspruch 5, wobei jede erste Seitenplatte (116) mit einer ersten Drahtnut (710) in einer lateralen Seite der ersten Seitenplatte (116) ausgestattet ist, die sich entlang einer Längsrichtung der ersten Seitenplatte (116) erstreckt, wobei eine periphere Seite der zweiten Montageplatte (120) mit einer zweiten Drahtnut (720) ausgestattet ist, die sich um einen Umfang der zweiten Montageplatte (120) erstreckt, und wobei die beiden ersten Drahtnuten (710) der zwei ersten Seitenplatten (116) und die zweite Drahtnut (720) verbunden sind, um eine geschlossene und dreidimensionale Ringnut zu bilden;
wobei der erste Dichtungsstreifen (610, 1410) in der zweiten Drahtnut (720) eingebettet ist und jeder zweite Dichtungsstreifen (620, 1420) in einem der ersten Drahtnuten (710) eingebettet ist;
wobei eine Innenwand des ersten Seitengehäuses (200) mit einer ersten vorstehenden Rippe (810) versehen ist, die mit der Ringnut ineinandergreift, wobei eine Innenwand des zweiten Seitengehäuses (300) mit einer weiteren ersten vorstehenden Rippe (810) versehen ist, die mit der Ringnut ineinandergreift, und die ersten vorstehenden Rippen (810) den ersten Dichtungsstreifen (610, 1410) in der zweiten Drahtnut (720) eindrücken.

7. Tragbares Schönheitsinstrument nach Anspruch 3, wobei die vordere Gehäusebaugruppe (40) ein Vordergehäuse (400) und ein ringförmiges Gehäuse (900) umfasst, wobei das ringförmige Gehäuse (900) angeordnet zwischen dem Vordergehäuse (400) und der zweiten Montageplatte (120) ist, um das Vordergehäuse (400) und die zweite Montageplatte (120) zu verbinden, und wobei eine Seite des Vordergehäuses (400) und/oder des ringförmigen Gehäuses (900) mit einer Vertiefung ausgestattet ist;
wobei das tragbare Schönheitsinstrument ferner umfasst:
ein zweites Dichtungselement (1000), das einen Spalt zwischen dem ringförmigen Gehäuse (900) und der zweiten Montageplatte (120) abdichtet; und
eine Verbindungsrippe (630, 1430), die zwischen dem ersten Dichtungsstreifen (610, 1410) und dem zweiten Dichtungselement (1000) und/oder zwischen den beiden zweiten Dichtungsstreifen (620, 1420) verbunden ist.

8. Tragbares Schönheitsinstrument nach Anspruch 1, ferner umfassend ein Bodengehäuse (1300), wobei die erste Montageplatte (110) eine erste Platte (115) und zwei erste Seitenplatten (116) umfasst, wobei jede erste Seitenplatte (116) mit jeweils einer der beiden gegenüberliegenden Seiten der ersten Platte (115) verbunden ist;
wobei das Bodengehäuse (1300) an einem Ende der ersten Montageplatte (110) installiert ist, das von der zweiten Montageplatte (120) abgewandt ist, wobei beide Enden des Bodengehäuses (1300) in einer Längsrichtung des Bodengehäuses (1300) jeweils bis zu einem der beiden ersten Seitenplatten (116) reichen und mit diesem in Anlage gebracht werden.

9. Tragbares Schönheitsinstrument nach Anspruch 8, ferner umfassend eine erste Tastenbaugruppe (3200), wobei der Mittelrahmen (100) ferner eine dritte Montageplatte (700) umfasst;
wobei die dritte Montageplatte (700) auf einer Seite der ersten Montageplatte (110) angeordnet ist, die von der zweiten Montageplatte (120) abgewandt ist, und wobei die erste Tastenbaugruppe (3200) auf der dritten Montageplatte (700) angeordnet ist und zumindest teilweise aus dem Bodengehäuse (1300) freiliegt.

10. Tragbares Schönheitsinstrument nach Anspruch 9, wobei die erste Tastenbaugruppe (3200) eine Tasten-Leiterplatte (3210), eine Taste (3220), ein Tastengehäuse (3230) mit halbtransparentem oberen Teil und ein lichtemittierendes Element (3240) umfasst; wobei die Tasten-Leiterplatte(3210) und das lichtemittierende Element (3240) mit der Verarbeitungsschaltung (L1) verbunden sind;
wobei das Tastengehäuse (3230) die Taste (3220) und das lichtemittierende Element (3240) abdeckt, und wobei die Taste (3220) und das lichtemittierende Element (3240) auf der Tasten-Leiterplatte(3210) montiert sind.

11. Tragbares Schönheitsinstrument nach Anspruch 10, wobei das Tastengehäuse (3230) umfasst:
eine Tastengehäuseabdeckung (3231), der integral lichtdurchlässig ist;und
ein Tastengehäusekörper (3232) mit einem lichtgeschirmten Umfang und einer Oberseite, die mit einem Lichtdurchgangsloch (3233) ausgestattet ist, das dem lichtemittierenden Element (3240) entspricht, wobei Licht, das vom lichtemittierenden Element (3240) emittiert wird, durch das Lichtdurchgangsloch (3233) hindurchtritt und von der Tastengehäuseabdeckung (3231) projiziert wird.

12. Tragbares Schönheitsinstrument nach Anspruch 10, wobei ein Boden des Tastengehäuses (3230) vorsteht, um eine zweite Säule (3238) zu bilden, und eine Rippenplatte (32300) in der zweiten Säule (3238) vorgesehen ist;
wobei die Taste (3220) in die zweite Säule (3238) eingesetzt ist und die Rippenplatte (32300) in der zweiten Säule (3238) in Kontakt mit dem Tastengehäuse (3230) steht, und wobei das Tastengehäuse (3230) oberhalb der Taste (3220) gedrückt werden kann, um die Taste (3220) zu betätigen;
oder,
wobei ein Boden des Tastengehäuses (3230) vorsteht, um eine dritte Säule (3239) zu bilden, eine Rippenplatte (32300) in der dritten Säule (3239) vorgesehen ist und die Rippenplatte (32300) verschiedene Kammern in der dritten Säule (3239) definiert; die Tasten-Leiterplatte(3210) mit mindestens zwei lichtemittierenden Elementen (3240) versehen ist, die Anzahl der Kammern am Boden der dritten Säule (3239) der Anzahl der lichtemittierenden Elemente (3240) auf der Tasten-Leiterplatte(3210) entspricht, jedes lichtemittierende Element (3240) in einer der Kammern angeordnet ist, und das von jedem lichtemittierenden Element (3240) emittierte Licht von einer entsprechenden Kammer projiziert wird.

13. Tragbares Schönheitsinstrument nach Anspruch 10, wobei die erste Tastenbaugruppe (3200) ein elastisches Element (2000) umfasst, das zwischen einem freien Ende des Tastengehäuses (3230) und dem Bodengehäuse (1300) verbunden ist;
eine Seite des Bodengehäuses (1300), die dem Tastengehäuse (3230) zugewandt ist, mit einer Vielzahl von ersten Säulen (1310) versehen ist, das elastische Element (2000) mit Öffnungen (2001) ausgestattet ist, und jede erste Säule (1310) in eine der Öffnungen (2001) eingesetzt ist;
oder,
wobei das tragbare Schönheitsinstrument ferner eine zweite Tastenbaugruppe (3330) umfasst, und die erste Tastenbaugruppe (3200) und die zweite Tastenbaugruppe (3330) auf derselben Tasten-Leiterplatte(3210) angeordnet sind;
wobei das Bodengehäuse (1300) mit einer ersten Durchgangsöffnung (1110) und einer zweiten Durchgangsöffnung (1120) ausgestattet ist, wobei die erste Tastenbaugruppe (3200) durch die erste Durchgangsöffnung (1110) hindurchragt und wobei die zweite Tastenbaugruppe (3330) durch die zweite Durchgangsöffnung (1120) hindurchragt.

14. Ladestation des tragbaren Schönheitsinstruments nach einem der Ansprüche 1 bis 13, wobei die Ladestation umfasst:
einen ersten Sitzkörper (91), umfassend eine Stützfläche (911) und eine erste Begrenzungsfläche (912), wobei die Stützfläche (911) an die erste Begrenzungsfläche (912) anstößt, um einen Winkel dazwischen zu definieren und eine Erhebung zu bilden;
eine Ladekomponente (L3), die auf der Stützfläche (911) angeordnet ist; und
einen zweiten Sitzkörper (93), der mit einer Seite der ersten Begrenzungsfläche (912) des ersten Sitzkörpers (91) verbunden ist, wobei der zweite Sitzkörper (93) eine zweite Begrenzungsfläche (913) umfasst, wobei die zweite Begrenzungsfläche (913) und die erste Begrenzungsfläche (912) einen Winkel dazwischen definieren und eine Vertiefung bilden;
wobei die zweite Begrenzungsfläche (913) dazu konfiguriert ist, die vordere Gehäusebaugruppe (40) und die Elektrode (500) des tragbaren Schönheitsinstruments in einer ersten Begrenzungsrichtung (01) zu begrenzen, wobei die Vertiefung zwischen der zweiten Begrenzungsfläche (913) und der ersten Begrenzungsfläche (912) dazu konfiguriert ist, eine Seitenfläche der vorderen Gehäusebaugruppe (40) zu stützen, wobei die erste Begrenzungsfläche (912) dazu konfiguriert ist, einen Verbindungsabschnitt zwischen der vorderen Gehäusebaugruppe (40) und dem hinteren Gehäuse (400B) des tragbaren Schönheitsinstruments in einer zweiten Begrenzungsrichtung (02) zu begrenzen, und wobei die jeweilige horizontale Komponenten der ersten Begrenzungsrichtung (01) und der zweiten Begrenzungsrichtung (02) zueinander entgegengesetzt sind.

15. Ladestation nach Anspruch 14, wobei die Stützfläche (911) gekrümmt ist; und/oder wobei die Ladestation ferner eine Desinfektionslampe (920) umfasst, die auf der zweiten Begrenzungsfläche (913) angeordnet ist, um Licht auszusenden und das tragbare Schönheitsinstrument zu desinfizieren.

## Revendications

1. Équipement de beauté portable, comprenant : un ensemble de boîtier avant (40), un boîtier arrière (400B), un cadre intermédiaire (100), une électrode (500) et un circuit de traitement (L1) ; le cadre intermédiaire (100) comprenant une première plaque de montage (110) s'étendant selon une première direction (10) ;
le boîtier arrière (400B) entourant au moins partiellement la première plaque de montage (110) et définissant un deuxième espace d'accueil (1600) en coopération avec la première plaque de montage (110) ;
l'électrode (500) étant au moins partiellement exposée à l'extérieur de l'ensemble de boîtier avant (40) ; et
le circuit de traitement (L1) étant disposé dans le deuxième espace d'accueil (1600) et connecté à l'électrode (500) pour entraîner l'électrode (500) à se décharger ;
le cadre intermédiaire (100) comprend en outre une deuxième plaque de montage (120) s'étendant dans une deuxième direction (20) perpendiculaire à la première direction (10) ; l'ensemble de boîtier avant (40) est situé sur un côté de la deuxième plaque de montage (120) éloigné du boîtier arrière (400B), **caractérisé en ce que** l'ensemble de boîtier avant (40) définit un troisième espace d'accueil (1700) en coopération avec la deuxième plaque de montage (120) pour accueillir l'électrode (500) ;
l'électrode (500) est également disposée dans le troisième espace d'accueil (1700).

2. Équipement de beauté portable selon la revendication 1, dans lequel,
le boîtier arrière (400B) comprend un premier boîtier latéral (200) et un deuxième boîtier latéral (300), le premier boîtier latéral (200) est installé sur un côté de la première plaque de montage (110), et le deuxième boîtier latéral (300) est installé sur le côté à l'opposé de la première plaque de montage (110) ;
le premier boîtier latéral (200), la première plaque de montage (110) et la deuxième plaque de montage (120) définissent de manière coopérative un premier espace d'accueil (1500) ; le deuxième boîtier latéral (300), la première plaque de montage (110) et la deuxième plaque de montage (120) définissent de manière coopérative le deuxième espace d'accueil (1600).

3. Équipement de beauté portable selon la revendication 2, comprenant en outre un premier élément d'étanchéité (600, 1400), dans lequel le premier élément d'étanchéité (600, 1400) comprend une première bande d'étanchéité (610, 1410) et deux deuxièmes bandes d'étanchéité (620, 1420), qui sont toutes intégrées ;
la première bande d'étanchéité (610, 1410) entoure au moins partiellement un côté périphérique de la deuxième plaque de montage (120) pour étancher un espace entre le premier boîtier latéral (200) et la deuxième plaque de montage (120), ainsi qu'un espace entre le deuxième boîtier latéral (300) et la deuxième plaque de montage (120) ;
chaque deuxième bande d'étanchéité (620, 1420) est configurée pour étancher l'un des espaces formés entre un premier côté latéral (111) de la première plaque de montage (110) et le premier boîtier latéral (200), entre le premier côté latéral (111) et le deuxième boîtier latéral (300), entre un deuxième côté latéral (112) de la première plaque de montage (110) à l'opposé du premier côté latéral (111) et le premier boîtier latéral (200), et entre le deuxième côté latéral (112) et le deuxième boîtier latéral (300).

4. Équipement de beauté portable selon la revendication 3, dans lequel chaque deuxième bande d'étanchéité (620, 1420) s'étend le long des côtés avant et arrière de l'un du premier côté latéral (111) et du deuxième côté latéral (112) de la première plaque de montage (110), et entre en contact avec à la fois le premier boîtier latéral (200) et le deuxième boîtier latéral (300) ;
dans lequel, chaque deuxième bande d'étanchéité (620, 1420) s'étend le long de l'une des surfaces opposées de la première plaque de montage (110) pour étancher l'un des espaces formés entre une surface de la première plaque de montage (110) et le premier boîtier latéral (200), et entre la surface à l'opposé de la première plaque de montage (110) et le deuxième boîtier latéral (300).

5. Équipement de beauté portable selon la revendication 3, dans lequel la première plaque de montage (110) comprend une première plaque (115) et deux premières plaques latérales (116), chaque première plaque latérale (116) est reliée à l'un des deux côtés opposés de la première plaque (115), chaque première plaque latérale (116) présente une largeur supérieure à l'épaisseur de la première plaque (115), chaque deuxième bande d'étanchéité (620, 1420) est disposée autour du bord de l'une des premières plaques latérales (116) ;
les extrémités des deux premières plaques latérales (116) adjacentes à la deuxième plaque de montage (120) divisent la première bande d'étanchéité (610, 1410) en deux parties symétriques, chaque partie de la première bande d'étanchéité (610, 1410) s'étendant d'une des premières plaques latérales (116) à l'l'un.

6. Équipement de beauté portable selon la revendication 5, dans lequel chaque première plaque latérale (116) est pourvue d'un premier canal de fil (710) dans un côté latéral de la première plaque latérale (116) et s'étend selon une direction de longueur de la première plaque latérale (116), un côté périphérique de la deuxième plaque de montage (120) est pourvue d'un deuxième canal de fil (720) s'étendant autour d'une circonférence de la deuxième plaque de montage (120), et deux premiers canaux de fil (710) des deux premières plaques latérales (116) et le deuxième canal de fil (720) sont en communication pour former un canal annulaire fermé et tridimensionnel ;
la première bande d'étanchéité (610, 1410) est encastrée dans le deuxième canal de fil (720), et chaque deuxième bande d'étanchéité (620, 1420) est encastrée dans l'un des premiers canaux de fil (710) ;
dans lequel une paroi interne du premier boîtier latéral (200) est pourvu d'une première nervure saillante (810) qui est emboîtée avec le canal annulaire, une paroi interne du deuxième boîtier latéral (300) est pourvue d'une autre première nervure saillante (810) qui est emboîtée avec le canal annulaire, et les premières nervures saillantes (810) pressent la première bande d'étanchéité (610, 1410) dans le deuxième canal de fil (720).

7. Équipement de beauté portable selon la revendication 3, dans lequel l'ensemble de boîtier avant (40) comprend un boîtier avant (400) et un boîtier annulaire (900), le boîtier annulaire (900) étant situé entre le boîtier avant (400) et la deuxième plaque de montage (120) pour relier le boîtier avant (400) et la deuxième plaque de montage (120), et un côté du boîtier avant (400) et/ou du boîtier annulaire (900) est pourvu d'un renfoncement ;
dans lequel l'équipement de beauté portable comprend en outre :
un deuxième élément d'étanchéité (1000), qui étanche un espace entre le boîtier annulaire (900) et la deuxième plaque de montage (120) ; et
une nervure de liaison (630, 1430), reliée entre la première bande d'étanchéité (610, 1410) et le deuxième élément d'étanchéité (1000), et/ou reliée entre les deux deuxièmes bandes d'étanchéité (620, 1420).

8. Équipement de beauté portable selon la revendication 1, comprenant en outre un boîtier inférieur (1300), dans lequel la première plaque de montage (110) comprend une première plaque (115) et deux premières plaques latérales (116), chaque première plaque latérale (116) étant reliée à l'un des deux côtés opposés de la première plaque (115) ;
le boîtier inférieur (1300) est installé à une extrémité de la première plaque de montage (110) éloignée de la deuxième plaque de montage (120), et deux extrémités du boîtier inférieur (1300) dans une direction de longueur du boîtier inférieur (1300) s'étendent respectivement pour s'appuyer contre l'une des deux premières plaques latérales (116).

9. Équipement de beauté portable selon la revendication 8, comprenant en outre un premier ensemble de bouton (3200), dans lequel le cadre intermédiaire (100) comprend en outre une troisième plaque de montage (700) ;
la troisième plaque de montage (700) est disposée sur un côté de la première plaque de montage (110) éloigné de la deuxième plaque de montage (120), et le premier ensemble de bouton (3200) est disposé sur la troisième plaque de montage (700) et est au moins partiellement exposé à l'extérieur du boîtier inférieur (1300).

10. Équipement de beauté portable selon la revendication 9, dans lequel le premier ensemble de bouton (3200) comprend une carte de circuit imprimé de bouton (3210), une touche (3220), un boîtier de touche (3230) avec un somment semi-transparent, et un élément lumineux (3240) ; la carte de circuit imprimé de bouton (3210) et l'élément lumineux (3240) sont connectés au circuit de traitement (L1) ;
le boîtier de touche (3230) recouvre la touche (3220) et l'élément lumineux (3240), et la touche (3220) et l'élément lumineux (3240) sont montés sur la carte de circuit imprimé de bouton (3210).

11. Équipement de beauté portable selon la revendication 10, dans lequel le boîtier de touches (3230) comprend :
un couvercle de boîtier de touche (3231), qui est intégralement pénétrable à la lumière ; et
un corps de boîtier de touche (3232), avec un périmètre pare-lumière et un sommet pourvu d'un trou pénétrable à la lumière (3233) correspondant à l'élément lumineux (3240), dans lequel la lumière émise par l'élément lumineux (3240) passe par le trou pénétrable à la lumière (3233) et est projetée depuis le couvercle du boîtier de touche (3231).

12. Équipement de beauté portable selon la revendication 10, dans lequel un fond du boîtier de touche (3230) fait saille pour former une deuxième colonne (3238), et une plaque de nervure (32300) est prévue dans la deuxième colonne (3238) ;
la touche (3220) est insérée dans la deuxième colonne (3238), la plaque de nervure (32300) dans la deuxième colonne (3238) entre en contact avec le boîtier de touche (3230), et le boîtier de touche (3230) au-dessus de la touche (3220) est capable être appuyé pour activer la touche (3220) ;
ou,
dans lequel un fond du boîtier de touche (3230) fait saille pour former une troisième colonne (3239), une plaque de nervure (32300) est prévue dans la troisième colonne (3239), et la plaque de nervure (32300) définit différents compartiments dans la troisième colonne (3239) ; la carte de circuit imprimé de bouton (3210) est pourvue d'au moins deux élément lumineux (3240), le nombre des compartiments à un fond de la troisième colonne (3239) correspond au nombre des éléments lumineux (3240) sur la carte de circuit imprimé de bouton (3210), chaque élément lumineux (3240) est placé dans un des compartiments, et la lumière émise par chaque élément lumineux (3240) est projetée depuis un compartiment correspondant.

13. Équipement de beauté portable selon la revendication 10, dans lequel le premier ensemble de bouton (3200) comprend un élément élastique (2000) relié entre une extrémité libre du boîtier de touche (3230) et le boîtier inférieur (1300) ;
un côté du boîtier inférieur (1300) faisant face au boîtier de touche (3230) est pourvu d'une pluralité de premières colonnes (1310), l'élément élastique (2000) est pourvu d'ouvertures (2001), et chaque première colonne (1310) est insérée dans l'une des ouvertures (2001) ;
ou,
dans lequel l'équipement de beauté portable comprend en outre un deuxième ensemble de bouton (3330), et le premier ensemble de bouton (3200) et le deuxième ensemble de bouton (3330) sont disposés sur une même carte de circuit imprimé de bouton (3210) ;
le boîtier inférieur (1300) est pourvu d'un premier trou traversant (1110) et un deuxième trou traversant (1120), le premier ensemble de bouton (3200) dépasse à travers le premier trou traversant (1110), et le deuxième ensemble de bouton (3330) dépasse à travers le deuxième trou traversant (1120).

14. Dock de recharge de l'équipement de beauté portable selon l'une quelconque des revendications 1 à 13, dans lequel le dock de recharge comprend :
un premier corps de siège (91) comprenant une surface de support (911) et une première surface de limitation (912), la surface de support (911) étant adjacente à la première surface de limitation (912) pour définir un angle entre elles et former une projection ;
un composant de recharge (L3) disposé sur la surface de support (911) ; et
un deuxième corps de siège (93) relié à un côté de la première surface de limitation (912) du premier corps de siège (91), le deuxième corps de siège (93) comprenant une deuxième surface de limitation (913), la deuxième surface de limitation (913) et la première surface de limitation (912) définissant un angle entre elles et une dépression ;
dans lequel la deuxième surface de limitation (913) est configurée pour limiter l'ensemble de boîtier avant (40) et l'électrode (500) de l'équipement de beauté portable dans une première direction de limitation (01), la dépression entre la deuxième surface de limitation (913) et la première surface de limitation (912) est configurée pour supporter une surface latérale de l'ensemble de boîtier avant (40), la première surface de limitation (912) est configurée pour limiter une portion de liaison entre l'ensemble de boîtier avant (40) et le boîtier arrière (400B) de l'équipement de beauté portable dans une deuxième direction de limitation (02), et les composants horizontaux respectifs de la première direction de limitation (01) et de la deuxième direction de limitation (02) sont opposés l'un à l'autre.

15. Dock de recharge de la revendication 14, dans lequel la surface de support (911) est courbée ; et/ou, dans lequel le dock de recharge comprend en outre une lampe de désinfection (920) disposée sur la deuxième surface de limitation (913) pour émettre de la lumière afin de désinfecter l'équipement de beauté portable.
